# EUROPEAN PATENT SPECIFICATION

(11) **EP 1 704 164 B1**
(45) Date of publication and mention of the grant of the patent: **28.12.2011**
(21) Application number: 04805919.0
(22) Date of filing: 03.12.2004
(51) Int. Cl.: C07K 14/54, C07K 14/765

(54) **INTERLEUKIN-11 FUSION PROTEINS**
INTERLEUKIN-11-FUSIONSPROTEINE
PROTEINES DE FUSION INTERLEUKINE-11

(30) Priority: 03.12.2003 EP 03027770
(43) Date of publication of application: 27.09.2006
(73) Proprietor: Kröz, Monika, 88499 Althein (DE); Weimer, Thomas, 35075 Gladenbach (DE); Hauser, Hans-Peter, 35041 Marburg (DE); Dickneite, Gerhard, 35043 Marburg (DE)
(72) Inventor: KRÖZ, Monika, 88499 Althein (DE); DICKNEITE, Gerhard, 35002 Marburg (DE); HAUSER, Hans-Peter, 35041 Marburg (DE); WEIMER, Thomas, 35002 Marburg (DE); SLEEP, Darrell, West Bridgford, Nottingham NG2 5DS (GB)
(74) Representative: Hauser, Hans-Peter
(86) International application number: PCT/GB2004/005091
(87) International publication number: WO 2005/054286

(56) References cited:
- US-A1- 2003 125 247
- US-B1- 6 540 993

### SEQUENCE LISTING

<110> Delta Biotechnology Limited
   <120> Interleukin-11 fusion protein
<130> DELF P29719EP
<140> 03027770.1
   <141> 13/12/03
<160> 20
<170> SeqWin99
<210> 1
   <211> 48
   <212> DNA
   <213> Artificial Sequence
<220>
   <223> Oligonucleotide Linker VC053
<400> 1
   gatctttgga taagagagac gctcacaagt ccgaagtcgc tcaccggt 48
<210> 2
   <211> 50
   <212> DNA
   <213> Artificial Sequence
<220>
   <223> Oligonucleotide Linker VC054
<400> 2
   ccttgaaccg gtgagcgact tcggacttgt gagcgtctct cttatccaaa 50
<210> 3
   <211> 48
   <212> DNA
   <213> Artificial Sequence
<220>
   <223> Oligonucleotide Linker VC055
<400> 3
   gatctttgga taagagagac gctcacaagt ccgaagtcgc tcatcgat 48
<210> 4
   <211> 50
   <212> DNA
   <213> Artificial Sequence
<220>
   <223> Oligonucleotide Linker VC056
<400> 4
   ccttgaatcg atgagcgact tcggacttgt gagcgtctct cttatccaaa 50
<210> 5
   <211> 86
   <212> DNA
   <213> Artificial Sequence
<220>
   <223> Oligonucleotide Linker VC057
<400> 5
   tcaaggacct aggtgaggaa aacttcaagg ctttggtctt gatcgctttc gctcaatact 60
   tgcaacaatg tccattcgaa gatcac 86
<210> 6
   <211> 80
   <212> DNA
   <213> Artificial Sequence
<220>
   <223> Oligonucleotide Linker VC058
<400> 6
   gtgatcttcg aatggacatt gttgcaagta ttgagcgaaa gcgatcaaga ccaaagcctt 60
   gaagttttcc tcacctaggt 80
<210> 7
   <211> 54
   <212> DNA
   <213> Oligonucleotide Primer CF59
<400> 7
   cgatagatct ttggataaga gagggccacc acctggcccc cctcgagttt cccc 54
<210> 8
   <211> 5
   <212> PRT
   <213> Artificial Sequence
<220>
   <223> Polypeptide linker SEQ ID 8
<400> 8
   Gly Gly Gly Gly Ser
   1 5
<210> 9
   <211> 4
   <212> PRT
   <213> Artificial Sequence
<220>
   <223> Polypeptide linker SEQ ID 9
<400> 9
   Gly Gly Gly Ser
   1
<210> 10
   <211> 17
   <212> PRT
   <213> Stanniocalcin signal peptide sequence
<400> 10
   Met Leu Gln Asn Ser Ala Val Leu Leu Leu Leu Val Ile Ser Ala Ser
   1 5 10 15
   Ala
<210> 11
   <211> 22
   <212> PRT
   <213> Consensus signal sequence
<400> 11
   Met Pro Thr Trp Ala Trp Trp Leu Phe Leu Val Leu Leu Leu Ala Leu
   1 5 10 15
   Trp Ala Pro Ala Arg Gly
   20
<210> 12
   <211> 66
   <212> DNA
   <213> Artificial Sequence
<220>
   <223> Oligonucleotide Primer CF60
<400> 12
   ggccatcgat gagcgacttc ggacttgtga gcgtccagcc gagtcttcag cagcagcagt 60
   cccctc 66
<210> 13
   <211> 50
   <212> DNA
   <213> Artificial Sequence
<220>
   <223> Oligonucleotide Primer CF61
<400> 13
   ccggccttag gcttacctgg gccaccacct ggcccccctc gagtttcccc 50
<210> 14
   <211> 45
   <212> DNA
   <213> Artificial Sequence
<220>
   <223> Oligonucleotide Primer CF62
<400> 14
   ggccaagctt attacagccg agtcttcagc agcagcagtc ccctc 45
<210> 15
   <211> 2358
   <212> DNA
   <213> Artificial Sequence
<220>
   <223> N-terminal IL11-albumin fusion
<400> 15
   atgaagtggg ttttcatcgt ctccattttg ttcttgttct cctctgctta ctctagatct   60
   ttggataaga gagggccacc acctggcccc cctcgagttt ccccagaccc tcgggccgag  120
   ctggacagca ccgtgctcct gacccgctct ctcctggcgg acacgcggca gctggctgca  180
   cagctgaggg acaaattccc agctgacggg gaccacaacc tggattccct gcccaccctg  240
   gccatgagtg cgggggcact gggagctcta cagctcccag gtgtgctgac aaggctgcga  300
   gcggacctac tgtcctacct gcggcacgtg cagtggctgc gccgggcagg tggctcttcc  360
   ctgaagaccc tggagcccga gctgggcacc ctgcaggccc gactggaccg gctgctgcgc  420
   cggctgcagc tcctgatgtc ccgcctggcc ctgccccagc cacccccgga cccgccggcg  480
   cccccgctgg cgcccccctc ctcagcctgg gggggcatca gggccgccca cgccatcctg  540
   ggggggctgc acctgacact tgactgggcc gtgaggggac tgctgctgct gaagactcgg  600
   ctggacgctc acaagtccga agtcgctcat cgattcaagg acctaggtga ggaaaacttc  660
   aaggctttgg tcttgatcgc tttcgctcaa tacttgcaac aatgtccatt cgaagatcac  720
   gtcaagttgg tcaacgaagt taccgaattc gctaagactt gtgttgctga cgaatctgct  780
   gaaaactgtg acaagtcctt gcacaccttg ttcggtgata agttgtgtac tgttgctacc  840
   ttgagagaaa cctacggtga aatggctgac tgttgtgcta agcaagaacc agaaagaaac  900
   gaatgtttct tgcaacacaa ggacgacaac ccaaacttgc caagattggt tagaccagaa  960
   gttgacgtca tgtgtactgc tttccacgac aacgaagaaa ccttcttgaa gaagtacttg 1020
   tacgaaattg ctagaagaca cccatacttc tacgctccag aattgttgtt cttcgctaag 1080
   agatacaagg ctgctttcac cgaatgttgt caagctgctg ataaggctgc ttgtttgttg 1140
   ccaaagttgg atgaattgag agacgaaggt aaggcttctt ccgctaagca aagattgaag 1200
   tgtgcttcct tgcaaaagtt cggtgaaaga gctttcaagg cttgggctgt cgctagattg 1260
   tctcaaagat tcccaaaggc tgaattcgct gaagtttcta agttggttac tgacttgact 1320
   aaggttcaca ctgaatgttg tcacggtgac ttgttggaat gtgctgatga cagagctgac 1380
   ttggctaagt acatctgtga aaaccaagac tctatctctt ccaagttgaa ggaatgttgt 1440
   gaaaagccat tgttggaaaa gtctcactgt attgctgaag ttgaaaacga tgaaatgcca 1500
   gctgacttgc catctttggc tgctgacttc gttgaatcta aggacgtttg taagaactac 1560
   gctgaagcta aggacgtctt cttgggtatg ttcttgtacg aatacgctag aagacaccca 1620
   gactactccg ttgtcttgtt gttgagattg gctaagacct acgaaactac cttggaaaag 1680
   tgttgtgctg ctgctgaccc acacgaatgt tacgctaagg ttttcgatga attcaagcca 1740
   ttggtcgaag aaccacaaaa cttgatcaag caaaactgtg aattgttcga acaattgggt 1800
   gaatacaagt tccaaaacgc tttgttggtt agatacacta agaaggtccc acaagtctcc 1860
   accccaactt tggttgaagt ctctagaaac ttgggtaagg tcggttctaa gtgttgtaag 1920
   cacccagaag ctaagagaat gccatgtgct gaagattact tgtccgtcgt tttgaaccaa 1980
   ttgtgtgttt tgcacgaaaa gaccccagtc tctgatagag tcaccaagtg ttgtactgaa 2040
   tctttggtta acagaagacc atgtttctct gctttggaag tcgacgaaac ttacgttcca 2100
   aaggaattca acgctgaaac tttcaccttc cacgctgata tctgtacctt gtccgaaaag 2160
   gaaagacaaa ttaagaagca aactgctttg gttgaattgg tcaagcacaa gccaaaggct 2220
   actaaggaac aattgaaggc tgtcatggat gatttcgctg ctttcgttga aaagtgttgt 2280
   aaggctgatg ataaggaaac ttgtttcgct gaagaaggta agaagttggt cgctgcttcc 2340
   caagctgctt tgggtttg                                              2358
<210> 16
   <211> 786
   <212> PRT
   <213> Artificial Sequence
<220>
   <223> N-terminal IL11-albumin fusion
<400> 16
   Met Lys Trp Val Phe Ile Val Ser Ile Leu Phe Leu Phe Ser Ser Ala
   1               5                   10                  15
   Tyr Ser Arg Ser Leu Asp Lys Arg Gly Pro Pro Pro Gly Pro Pro Arg
               20                  25                  30
   Val Ser Pro Asp Pro Arg Ala Glu Leu Asp Ser Thr Val Leu Leu Thr
           35                  40                  45
   Arg Ser Leu Leu Ala Asp Thr Arg Gln Leu Ala Ala Gln Leu Arg Asp
       50                  55                  60
   Lys Phe Pro Ala Asp Gly Asp His Asn Leu Asp Ser Leu Pro Thr Leu
   65                  70                  75                  80
   Ala Met Ser Ala Gly Ala Leu Gly Ala Leu Gln Leu Pro Gly Val Leu
                   85                  90                  95
   Thr Arg Leu Arg Ala Asp Leu Leu Ser Tyr Leu Arg His Val Gln Trp
               100                 105                 110
   Leu Arg Arg Ala Gly Gly Ser Ser Leu Lys Thr Leu Glu Pro Glu Leu
           115                 120                 125
   Gly Thr Leu Gln Ala Arg Leu Asp Arg Leu Leu Arg Arg Leu Gln Leu
       130                 135                 140
   Leu Met Ser Arg Leu Ala Leu Pro Gln Pro Pro Pro Asp Pro Pro Ala
   145                 150                 155                 160
   Pro Pro Leu Ala Pro Pro Ser Ser Ala Trp Gly Gly Ile Arg Ala Ala
                   165                 170                 175
   His Ala Ile Leu Gly Gly Leu His Leu Thr Leu Asp Trp Ala Val Arg
               180                 185                 190
   Gly Leu Leu Leu Leu Lys Thr Arg Leu Asp Ala His Lys Ser Glu Val
           195                 200                 205
   Ala His Arg Phe Lys Asp Leu Gly Glu Glu Asn Phe Lys Ala Leu Val
       210                 215                 220
   Leu Ile Ala Phe Ala Gln Tyr Leu Gln Gln Cys Pro Phe Glu Asp His
   225                 230                 235                 240
   Val Lys Leu Val Asn Glu Val Thr Glu Phe Ala Lys Thr Cys Val Ala
                   245                 250                 255
   Asp Glu Ser Ala Glu Asn Cys Asp Lys Ser Leu His Thr Leu Phe Gly
               260                 265                 270
   Asp Lys Leu Cys Thr Val Ala Thr Leu Arg Glu Thr Tyr Gly Glu Met
           275                 280                 285
   Ala Asp Cys Cys Ala Lys Gln Glu Pro Glu Arg Asn Glu Cys Phe Leu
       290                 295                 300
   Gln His Lys Asp Asp Asn Pro Asn Leu Pro Arg Leu Val Arg Pro Glu
   305                 310                 315                 320
   Val Asp Val Met Cys Thr Ala Phe His Asp Asn Glu Glu Thr Phe Leu
                   325                 330                 335
   Lys Lys Tyr Leu Tyr Glu Ile Ala Arg Arg His Pro Tyr Phe Tyr Ala
               340                 345                 350
   Pro Glu Leu Leu Phe Phe Ala Lys Arg Tyr Lys Ala Ala Phe Thr Glu
           355                 360                 365
   Cys Cys Gln Ala Ala Asp Lys Ala Ala Cys Leu Leu Pro Lys Leu Asp
   370 375 380
   Glu Leu Arg Asp Glu Gly Lys Ala Ser Ser Ala Lys Gln Arg Leu Lys
   385 390 395 400
   Cys Ala Ser Leu Gln Lys Phe Gly Glu Arg Ala Phe Lys Ala Trp Ala
   405 410 415
   Val Ala Arg Leu Ser Gln Arg Phe Pro Lys Ala Glu Phe Ala Glu Val
   420 425 430
   Ser Lys Leu Val Thr Asp Leu Thr Lys Val His Thr Glu Cys Cys His
   435 440 445
   Gly Asp Leu Leu Glu Cys Ala Asp Asp Arg Ala Asp Leu Ala Lys Tyr
   450 455 460
   Ile Cys Glu Asn Gln Asp Ser Ile Ser Ser Lys Leu Lys Glu Cys Cys
   465 470 475 480
   Glu Lys Pro Leu Leu Glu Lys Ser His Cys Ile Ala Glu Val Glu Asn
   485 490 495
   Asp Glu Met Pro Ala Asp Leu Pro Ser Leu Ala Ala Asp Phe Val Glu
   500 505 510
   Ser Lys Asp Val Cys Lys Asn Tyr Ala Glu Ala Lys Asp Val Phe Leu
   515 520 525
   Gly Met Phe Leu Tyr Glu Tyr Ala Arg Arg His Pro Asp Tyr Ser Val
   530 535 540
   Val Leu Leu Leu Arg Leu Ala Lys Thr Tyr Glu Thr Thr Leu Glu Lys
   545 550 555 560
   Cys Cys Ala Ala Ala Asp Pro His Glu Cys Tyr Ala Lys Val Phe Asp
   565 570 575
   Glu Phe Lys Pro Leu Val Glu Glu Pro Gln Asn Leu Ile Lys Gln Asn
   580 585 590
   Cys Glu Leu Phe Glu Gln Leu Gly Glu Tyr Lys Phe Gln Asn Ala Leu
   595 600 605
   Leu Val Arg Tyr Thr Lys Lys Val Pro Gln Val Ser Thr Pro Thr Leu
   610 615 620
   Val Glu Val Ser Arg Asn Leu Gly Lys Val Gly Ser Lys Cys Cys Lys
   625 630 635 640
   His Pro Glu Ala Lys Arg Met Pro Cys Ala Glu Asp Tyr Leu Ser Val
   645 650 655
   Val Leu Asn Gln Leu Cys Val Leu His Glu Lys Thr Pro Val Ser Asp
   660 665 670
   Arg Val Thr Lys Cys Cys Thr Glu Ser Leu Val Asn Arg Arg Pro Cys
   675 680 685
   Phe Ser Ala Leu Glu Val Asp Glu Thr Tyr Val Pro Lys Glu Phe Asn
   690 695 700
   Ala Glu Thr Phe Thr Phe His Ala Asp Ile Cys Thr Leu Ser Glu Lys
   705 710 715 720
   Glu Arg Gln Ile Lys Lys Gln Thr Ala Leu Val Glu Leu Val Lys His
   725 730 735
   Lys Pro Lys Ala Thr Lys Glu Gln Leu Lys Ala Val Met Asp Asp Phe
   740 745 750
   Ala Ala Phe Val Glu Lys Cys Cys Lys Ala Asp Asp Lys Glu Thr Cys
   755 760 765
   Phe Ala Glu Glu Gly Lys Lys Leu Val Ala Ala Ser Gln Ala Ala Leu
   770 775 780
   Gly Leu
   785
<210> 17
   <211> 762
   <212> PRT
   <213> Artificial Sequence
<220>
   <223> Mature N-terminal IL11-albumin fusion
<400> 17
   Gly Pro Pro Pro Gly Pro Pro Arg Val Ser Pro Asp Pro Arg Ala Glu
   1               5                   10                  15
   Leu Asp Ser Thr Val Leu Leu Thr Arg Ser Leu Leu Ala Asp Thr Arg
               20                  25                  30
   Gln Leu Ala Ala Gln Leu Arg Asp Lys Phe Pro Ala Asp Gly Asp His
           35                  40                  45
   Asn Leu Asp Ser Leu Pro Thr Leu Ala Met Ser Ala Gly Ala Leu Gly
       50                  55                  60
   Ala Leu Gln Leu Pro Gly Val Leu Thr Arg Leu Arg Ala Asp Leu Leu
   65                  70                  75                  80
   Ser Tyr Leu Arg His Val Gln Trp Leu Arg Arg Ala Gly Gly Ser Ser
                   85                  90                  95
   Leu Lys Thr Leu Glu Pro Glu Leu Gly Thr Leu Gln Ala Arg Leu Asp
               100                 105                 110
   Arg Leu Leu Arg Arg Leu Gln Leu Leu Met Ser Arg Leu Ala Leu Pro
           115                 120                 125
   Gln Pro Pro Pro Asp Pro Pro Ala Pro Pro Leu Ala Pro Pro Ser Ser
       130                 135                 140
   Ala Trp Gly Gly Ile Arg Ala Ala His Ala Ile Leu Gly Gly Leu His
   145                 150                 155                 160
   Leu Thr Leu Asp Trp Ala Val Arg Gly Leu Leu Leu Leu Lys Thr Arg
                   165                 170                 175
   Leu Asp Ala His Lys Ser Glu Val Ala His Arg Phe Lys Asp Leu Gly
               180                 185                 190
   Glu Glu Asn Phe Lys Ala Leu Val Leu Ile Ala Phe Ala Gln Tyr Leu
           195                 200                 205
   Gln Gln Cys Pro Phe Glu Asp His Val Lys Leu Val Asn Glu Val Thr
       210                 215                 220
   Glu Phe Ala Lys Thr Cys Val Ala Asp Glu Ser Ala Glu Asn Cys Asp
   225                 230                 235                 240
   Lys Ser Leu His Thr Leu Phe Gly Asp Lys Leu Cys Thr Val Ala Thr
                   245                 250                 255
   Leu Arg Glu Thr Tyr Gly Glu Met Ala Asp Cys Cys Ala Lys Gln Glu
               260                 265                 270
   Pro Glu Arg Asn Glu Cys Phe Leu Gln His Lys Asp Asp Asn Pro Asn
           275                 280                 285
   Leu Pro Arg Leu Val Arg Pro Glu Val Asp Val Met Cys Thr Ala Phe
       290                 295                 300
   His Asp Asn Glu Glu Thr Phe Leu Lys Lys Tyr Leu Tyr Glu Ile Ala
   305                 310                 315                 320
   Arg Arg His Pro Tyr Phe Tyr Ala Pro Glu Leu Leu Phe Phe Ala Lys
                   325                 330                 335
   Arg Tyr Lys Ala Ala Phe Thr Glu Cys Cys Gln Ala Ala Asp Lys Ala
               340                 345                 350
   Ala Cys Leu Leu Pro Lys Leu Asp Glu Leu Arg Asp Glu Gly Lys Ala
           355                 360                 365
   Ser Ser Ala Lys Gln Arg Leu Lys Cys Ala Ser Leu Gln Lys Phe Gly
       370                 375                 380
   Glu Arg Ala Phe Lys Ala Trp Ala Val Ala Arg Leu Ser Gln Arg Phe
   385                 390                 395                 400
   Pro Lys Ala Glu Phe Ala Glu Val Ser Lys Leu Val Thr Asp Leu Thr
                   405                 410                 415
   Lys Val His Thr Glu Cys Cys His Gly Asp Leu Leu Glu Cys Ala Asp
               420                 425                 430
   Asp Arg Ala Asp Leu Ala Lys Tyr Ile Cys Glu Asn Gln Asp Ser Ile
           435                 440                 445
   Ser Ser Lys Leu Lys Glu Cys Cys Glu Lys Pro Leu Leu Glu Lys Ser
       450                 455                 460
   His Cys Ile Ala Glu Val Glu Asn Asp Glu Met Pro Ala Asp Leu Pro
   465                 470                 475                 480
   Ser Leu Ala Ala Asp Phe Val Glu Ser Lys Asp Val Cys Lys Asn Tyr
                   485                 490                 495
   Ala Glu Ala Lys Asp Val Phe Leu Gly Met Phe Leu Tyr Glu Tyr Ala
               500                 505                 510
   Arg Arg His Pro Asp Tyr Ser Val Val Leu Leu Leu Arg Leu Ala Lys
           515                 520                 525
   Thr Tyr Glu Thr Thr Leu Glu Lys Cys Cys Ala Ala Ala Asp Pro His
       530                 535                 540
   Glu Cys Tyr Ala Lys Val Phe Asp Glu Phe Lys Pro Leu Val Glu Glu
   545                 550                 555                 560
   Pro Gln Asn Leu Ile Lys Gln Asn Cys Glu Leu Phe Glu Gln Leu Gly
                   565                 570                 575
   Glu Tyr Lys Phe Gln Asn Ala Leu Leu Val Arg Tyr Thr Lys Lys Val
               580                 585                 590
   Pro Gln Val Ser Thr Pro Thr Leu Val Glu Val Ser Arg Asn Leu Gly
           595                 600                 605
   Lys Val Gly Ser Lys Cys Cys Lys His Pro Glu Ala Lys Arg Met Pro
       610                 615                 620
   Cys Ala Glu Asp Tyr Leu Ser Val Val Leu Asn Gln Leu Cys Val Leu
   625                 630                 635                 640
   His Glu Lys Thr Pro Val Ser Asp Arg Val Thr Lys Cys Cys Thr Glu
                   645                 650                 655
   Ser Leu Val Asn Arg Arg Pro Cys Phe Ser Ala Leu Glu Val Asp Glu
               660                 665                 670
   Thr Tyr Val Pro Lys Glu Phe Asn Ala Glu Thr Phe Thr Phe His Ala
           675                 680                 685
   Asp Ile Cys Thr Leu Ser Glu Lys Glu Arg Gln Ile Lys Lys Gln Thr
       690                 695                 700
   Ala Leu Val Glu Leu Val Lys His Lys Pro Lys Ala Thr Lys Glu Gln
   705                 710                 715                 720
   Leu Lys Ala Val Met Asp Asp Phe Ala Ala Phe Val Glu Lys Cys Cys
                   725                 730                 735
   Lys Ala Asp Asp Lys Glu Thr Cys Phe Ala Glu Glu Gly Lys Lys Leu
               740                 745                 750
   Val Ala Ala Ser Gln Ala Ala Leu Gly Leu
           755                 760
<210> 18
   <211> 2361
   <212> DNA
   <213> Artificial Sequence
<220>
   <223> C-terminal albumin-IL11 fusion
<400> 18
   atgaagtggg taagctttat ttcccttctt tttctcttta gctcggctta ttccaggagc   60
   ttggataaaa gagatgcaca caagagtgag gttgctcatc ggtttaaaga tttgggagaa  120
   gaaaatttca aagccttggt gttgattgcc tttgctcagt atcttcagca gtgtccattt  180
   gaagatcatg taaaattagt gaatgaagta actgaatttg caaaaacatg tgttgctgat  240
   gagtcagctg aaaattgtga caaatcactt catacccttt ttggagacaa attatgcaca   300
   gttgcaactc ttcgtgaaac ctatggtgaa atggctgact gctgtgcaaa acaagaacct  360
   gagagaaatg aatgcttctt gcaacacaaa gatgacaacc caaacctccc ccgattggtg  420
   agaccagagg ttgatgtgat gtgcactgct tttcatgaca atgaagagac atttttgaaa  480
   aaatacttat atgaaattgc cagaagacat ccttactttt atgccccgga actccttttc  540
   tttgctaaaa ggtataaagc tgcttttaca gaatgttgcc aagctgctga taaagctgcc  600
   tgcctgttgc caaagctcga tgaacttcgg gatgaaggga aggcttcgtc tgccaaacag  660
   agactcaagt gtgccagtct ccaaaaattt ggagaaagag ctttcaaagc atgggcagta  720
   gctcgcctga gccagagatt tcccaaagct gagtttgcag aagtttccaa gttagtgaca  780
   gatcttacca aagtccacac ggaatgctgc catggagatc tgcttgaatg tgctgatgac  840
   agggcggacc ttgccaagta tatctgtgaa aatcaagatt cgatctccag taaactgaag  900
   gaatgctgtg aaaaacctct gttggaaaaa tcccactgca ttgccgaagt ggaaaatgat  960
   gagatgcctg ctgacttgcc ttcattagct gctgattttg ttgaaagtaa ggatgtttgc 1020
   aaaaactatg ctgaggcaaa ggatgtcttc ctgggcatgt ttttgtatga atatgcaaga 1080
   aggcatcctg attactctgt cgtgctgctg ctgagacttg ccaagacata tgaaaccact 1140
   ctagagaagt gctgtgccgc tgcagatcct catgaatgct atgccaaagt gttcgatgaa 1200
   tttaaacctc ttgtggaaga gcctcagaat ttaatcaaac aaaattgtga gctttttgag 1260
   cagcttggag agtacaaatt ccagaatgcg ctattagttc gttacaccaa gaaagtaccc 1320
   caagtgtcaa ctccaactct tgtagaggtc tcaagaaacc taggaaaagt gggcagcaaa 1380
   tgttgtaaac atcctgaagc aaaaagaatg ccctgtgcag aagactatct atccgtggtc 1440
   ctgaaccagt tatgtgtgtt gcatgagaaa acgccagtaa gtgacagagt caccaaatgc 1500
   tgcacagaat ccttggtgaa caggcgacca tgcttttcag ctctggaagt cgatgaaaca 1560
   tacgttccca aagagtttaa tgctgaaaca ttcaccttcc atgcagatat atgcacactt 1620
   tctgagaagg agagacaaat caagaaacaa actgcacttg ttgagctcgt gaaacacaag 1680
   cccaaggcaa caaaagagca actgaaagct gttatggatg atttcgcagc ttttgtagag 1740
   aagtgctgca aggctgacga taaggagacc tgctttgccg aggagggtaa aaaacttgtt 1800
   gctgcaagtc aagctgcctt aggcttacct gggccaccac ctggcccccc tcgagtttcc 1860
   ccagaccctc gggccgagct ggacagcacc gtgctcctga cccgctctct cctggcggac 1920
   acgcggcagc tggctgcaca gctgagggac aaattcccag ctgacgggga ccacaacctg 1980
   gattccctgc ccaccctggc catgagtgcg ggggcactgg gagctctaca gctcccaggt 2040
   gtgctgacaa ggctgcgagc ggacctactg tcctacctgc ggcacgtgca gtggctgcgc 2100
   cgggcaggtg gctcttccct gaagaccctg gagcccgagc tgggcaccct gcaggcccga 2160
   ctggaccggc tgctgcgccg gctgcagctc ctgatgtccc gcctggccct gccccagcca 2220
   cccccggacc cgccggcgcc cccgctggcg cccccctcct cagcctgggg gggcatcagg 2280
   gccgcccacg ccatcctggg ggggctgcac ctgacacttg actgggccgt gaggggactg 2340
   ctgctgctga agactcggct g                                           2361
<210> 19
   <211> 787
   <212> PRT
   <213> Artificial Sequence
<220>
   <223> C-terminal albumin-IL11 fusion
<400> 19
<210> 20
   <211> 763
   <212> PRT
   <213> Artificial Sequence
<220>
   <223> Mature C-terminal IL11-albumin fusion
<400> 20
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu Pro Gly Pro Pro Pro Gly Pro
               580                 585                 590
   Pro Arg Val Ser Pro Asp Pro Arg Ala Glu Leu Asp Ser Thr Val Leu
           595                 600                 605
   Leu Thr Arg Ser Leu Leu Ala Asp Thr Arg Gln Leu Ala Ala Gln Leu
       610                 615                 620
   Arg Asp Lys Phe Pro Ala Asp Gly Asp His Asn Leu Asp Ser Leu Pro
   625                 630                 635                 640
   Thr Leu Ala Met Ser Ala Gly Ala Leu Gly Ala Leu Gln Leu Pro Gly
                   645                 650                 655
   Val Leu Thr Arg Leu Arg Ala Asp Leu Leu Ser Tyr Leu Arg His Val
               660                 665                 670
   Gln Trp Leu Arg Arg Ala Gly Gly Ser Ser Leu Lys Thr Leu Glu Pro
           675                 680                 685
   Glu Leu Gly Thr Leu Gln Ala Arg Leu Asp Arg Leu Leu Arg Arg Leu
       690                 695                 700
   Gln Leu Leu Met Ser Arg Leu Ala Leu Pro Gln Pro Pro Pro Asp Pro
   705                 710                 715                 720
   Pro Ala Pro Pro Leu Ala Pro Pro Ser Ser Ala Trp Gly Gly Ile Arg
                   725                 730                 735
   Ala Ala His Ala Ile Leu Gly Gly Leu His Leu Thr Leu Asp Trp Ala
               740                 745                 750
   Val Arg Gly Leu Leu Leu Leu Lys Thr Arg Leu
           755                 760

## Description

### Field of the Invention

The invention relates to novel compositions for treatment or prophylaxis of thrombocytopenia, von Willebrand disease (vWD) or inflammatory diseases, such as inflammatory bowel disease (IBD).

### Background of the Invention

### Physiological function of IL-11

Interleukin eleven (IL-11) is a hematopoietic cytokine that promotes megakaryocytopoiesis and thrombocytopoiesis by stimulating the proliferation of primitive stem cells, multipotent and committed progenitor cells (synergistic with other hematopoietic growth factors like IL-3, IL-6, GM-CSF) resulting in megakaryocyte maturation and increased platelet production. Due to the activity described above it stimulates erythropoiesis as a side effect but shows little effect on neutrophil proliferation. It also has trophic effects on intestinal mucosa cells. Furthermore IL-11 induces the secretion of acute phase proteins (ferritin, haptoglobin, CRP, fibrinogen) by hepatocytes. It also shows antiinflammatory properties by inhibiting macrophage and T cell effector function. It inhibits the production of TNFα, IL-1β, IL-12, IL-6 and NO from activated macrophages in-vitro. Physiologically, IL-11 is produced by a variety of stromal cells, like fibroblasts, epithelial cells, chondrocytes and osteoblasts. In normal individuals it is generally undetectable in plasma. Degradation and elimination of IL-11 is yet poorly understood.

### Biochemical characteristics of IL-11

IL-11 is a 19 kDa polypeptide consisting of 178 amino acids (AA), plus 21 AA secretory leader sequence, which does not contain potential glycosylation residues, disulphide bonds or other posttranslational modifications, and has a close similarity to IL-6. It binds to a multimeric receptor complex which contains an IL-11 specific α-receptor subunit and a promiscuous β subunit (gp130).

Recombinant human IL-11 (Oprelvekin, Neumega^{®} by Genetics Institute/Wyeth) is a 2-178-interleukin-11 produced in *E*. *coli* lacking the amino terminal proline. This can be necessary to achieve secretion from the host cell but reportedly does not affect the activity of the cytokine.

### Chemotherapy-induced thrombocytopenia

Thrombocytopenia is a significant problem for patients receiving prolonged or aggressive chemotherapy for malignancies. For some chemotherapeutic agents, such as Carboplatin, it represents the predominant, dose-limiting toxicity and acts cumulatively.

Currently, platelet transfusion is the standard treatment for thrombocytopenia. However, platelet transfusions are expensive and associated with a significant risk of alloimmunisation and transmission of blood-borne diseases. Approximately 5 to 30% of platelet transfusions are associated with usually febrile, non-hemolytic reactions. Also, platelets are a limited resource with a shelf life of 5 days only. Thus, agents that promote platelet production are an attractive alternative to platelet transfusions for the prevention or treatment of thrombocytopenia. Recombinant human IL-11 (Oprelvekin, Neumega®) was approved in 1997 in the US for the secondary prophylaxis of chemotherapy-induced thrombocytopenia. Further potential indications include inflammatory bowel disease (IBD), Crohn's disease, colitis ulcerosa, psoriasis and von Willebrand's disease.

### Expected advantages of fusing IL11 to albumin

Safe and effective treatment of thrombocytopenia remains an unmet medical need. Recombinant human IL-11 (Oprelvekin, Neumega^{®}) has a very short half life of 6.9 hrs in humans and at the same time a narrow therapeutic window.

Prolongation of plasma-half-life and increased bioavailability through albumin-fusion are expected to result in an improved safety and efficacy profile. This means that therapeutic plasma levels can be achieved and maintained with lower doses and/or longer dose intervals, avoiding peak levels above the toxic threshold. Currently, Neumega^{®} is the only drug licensed for the treatment of chemotherapy-induced thrombocytopenia, representing a field with a high unmet medical need. Neumega^{®} shows a high incidence of toxic effects, such as edema and cardiovascular irregularities, combined with low efficacy especially in severe cases of thrombocytopenia.

### Summary of the Invention

The invention relates to proteins comprising IL-11 fused to albumin or fragments thereof. These fusion proteins are herein collectively referred to as "albumin fusion proteins of the invention." These fusion proteins of the invention exhibit extended *in vivo* half-life and/or extended therapeutic activity as compared to unfused IL11.

The invention encompasses therapeutic albumin fusion proteins, compositions, pharmaceutical compositions, formulations and kits. The invention also encompasses nucleic acid molecules encoding the albumin fusion proteins of the invention, as well as vectors containing these nucleic acids, host cells transformed with these nucleic acids and vectors, and methods of making the albumin fusion proteins of the invention using these nucleic acids, vectors, and/or host cells.

The invention also relates to compositions and methods for therapy and prevention of thrombocytopenia. The invention further relates to compositions and methods for antiinflammatory therapy and prevention. Also, the invention relates to compositions and methods for therapy and prevention of von Willebrand's disease.

### Brief Description of the Drawings

- Figure 1.: IL-11 plasma concentration after intravenous administration of rhIL-11 or C-terminal IL-11 -albumin fusion to rabbits
- Figure 2.: IL-11 plasma concentration after subcutaneous administration of rhIL-11 or C-terminal IL-11-albumin fusion to rabbits
- Figure 3.: IL-11 plasma concentration alter intravenous administration of rhIL-11 or N-terminal IL-11-albumin fusion to rats
- Figure 4.: IL-11 plasma concentration after subcutaneous administration of rhIL-11 or N-terTerminal IL-11-albumin fusion to rats
- Figure 5.: Course of platelet levels after treatment of naive rats with IL-11
- Figure 6.: Course of platelet levels after treatment of rats under chemotherapy with IL-11
- Figure 7.: Development of body weight (in % of baseline) after IL-11 application in a mouse model for IBD
- Figure 8.: Visual observation score (diarrhoea and gross rectal bleeding) after IL-11 application in a mouse model for IBD
- Figure 9.: Colon length after IL-11 application in a mouse model for IBD
- Figure 10.: Histological disease score after IL-11 application in a mouse model for IBD
- Figure 11: SDS gel and Western blots of various compounds of the invention.

### Detailed Description of the Invention

The present invention relates to fusion proteins comprising albumin coupled to IL-11. Such peptides include, but are not limited to, peptides binding to the gp130 receptor complex These peptides incldeIL-11, or fragments thereof, which have thrombopoietic or antiinflammatory properties.

The terms "protein" and "peptide" as used herein are non-limiting and include proteins and polypeptides as well as peptides.

Furthermore, chemical entities may be covalently attached to the fusion proteins of the invention or used in combinations to enhance a biological activity or to modulate a biological activity.

The albumin fusion proteins of the present invention are expected to prolong the half life of IL-11 *in vivo.* The *in vitro* or *in vivo* half-life of said albumin fused peptide/protein is extended 2-fold, or 5-fold, or more, over the half-life of the peptide/protein lacking the linked albumin. Furthermore, the albumin fusion proteins of the present invention are expected to reduce the frequency of the dosing schedule of the IL11 peptide. The dosing schedule frequency is reduced by at least one-quarter, or by at least one-half, or more, as compared to the frequency of the dosing schedule of the IL11 peptide lacking the linked albumin.

The albumin fusion proteins of the present invention are expected to prolong the shelf-life of the peptide, and/or stabilize the peptide and/or its activity in solution (or in a pharmaceutical composition) *in vitro* and/or *in vivo*. These albumin-fusion proteins, which may be therapeutic agents, are expected to reduce the need to formulate protein solutions with large excesses of carrier proteins (such as albumin, unfused) to prevent loss of proteins due to factors such as non-specific binding. An increased half life is defined as a half-life that is at least 2 times higher (preferably at least 5, 10, 20 or 30 times higher) than that of the unfused IL-11 compound, when measured over the first 24 hours after sub-cutaneous injection according to Example 4 below, in male Wistar rats aged 6 months.

The present invention also encompasses nucleic acid molecules encoding the albumin fusion proteins as well as vectors containing these nucleic acids, host cells transformed with these nucleic acids vectors, and methods of making the albumin fusion proteins of the invention using these nucleic acids, vectors, and/or host cells. The present invention further includes transgenic organisms modified to contain the nucleic acid molecules of the invention, optionally modified to express the albumin fusion proteins encoded by the nucleic acid molecules.

The present invention also encompasses pharmaceutical formulations comprising an albumin fusion protein of the invention and a pharmaceutically acceptable diluent or carrier. Such formulations may be in a kit or container. Such kit or container may be packaged with instructions pertaining to the extended shelf-life of the protein. Such formulations may be used in methods of preventing, treating, ameliorating thrombocytopenia or inflammatory diseases, such as inflammatory bowel disease, psoriasis, rheumatoid arthritis, von Willebrand's disease, etc., or a related disorder in a patient, such as a mammal, or a human, comprising the step of administering the pharmaceutical formulation to the patient

The invention also encompasses a method for potentially minimizing side effects (e.g., injection site reaction, increase of plasma volume, arrhythmia, headache, nausea, fever, rash, asthenia, diarrhoea, dizziness, allergic reactions) associated with the treatment of a mammal with cytokines in moderately higher concentrations comprising administering an albumin-fused cytokine of the invention to said mammal.

The present invention encompasses a method of preventing, treading or ameliorating thrombocytopenia or inflammatory diseases, such as inflammatory bowel disease, psoriasis, rheumatoid arthritis, etc., comprising administering to a mammal, in which such prevention treatment or amelioration is desired, an albumin fusion protein of the invention that comprises a IL-11 peptide/protein (or fragment thereof) in an amount effective to treat, prevent or ameliorate the disease or disorder. In the present invention, the IL-11 is also called the "therapeutic protein".

The present invention encompasses albumin fusion proteins comprising IL-11 (including fragments thereof) fused to albumin or multiple copies of albumin (including fragments thereof).

The present invention also encompasses a method for extending the half-life of IL-11 in a mammal. The method entails linking IL-11 to an albumin to form albumin-fused IL-11 and administering the albumin-fused IL-11 to a mammal. Typically, the half-life of the albumin-fused IL-11 may be extended by at least 2-fold, 5-fold, 10-fold, 20-fold, 30-fold, 40-fold or at least 50-fold over the half-life of IL-11 lacking the linked albumin.

Exemplified herein are fusion proteins comprising albumin fused to IL-11. The present invention also includes an improved method of manufacturing a therapeutic moiety as compared to what is available in the art. For example, the present invention provides an enhanced means of manufacturing a protein with the active moiety IL-11 Various aspects of the present invention are discussed in further detail below

### Albumin

The terms human serum albumin (HSA) and human albumin (HA) are used interchangeably herein. The terms, "albumin and "serum albumin" are broader, and encompass human serum albumin (and fragments thereof) as well as albumin from other species (and fragments thereof).

As used herein, "albumin" refers collectively to albumin protein or amino acid sequence, or an albumin fragment having one or more functional activities (e.g., biological activities) of albumin. In particular, "albumin" refers to human albumin or fragments thereof (see EP 201 239, EP 322 094 and WO 97/24445) especially the mature form of human albumin as shown in Table 1 and SEQ ID NO:18 of WO 03/066824 and WO 01/79480.

In particular, the albumin fusion proteins of the invention may include naturally occurring polymorphic variants of human albumin and fragments of human albumin.

### Albumin Fusion Proteins

The present invention relates generally to albumin fusion proteins and methods of treating, preventing or ameliorating diseases or disorders. As used herein, "albumin fusion protein" refers to a protein formed by the fusion of at least one molecule of albumin (or a fragment thereof) to at least one molecule of an IL11 protein (or fragment thereof). An albumin fusion protein of the invention comprises at least a fragment of an IL11 protein and at least a fragment of human serum albumin, which are associated with one another, such as by genetic fusion (i.e., the albumin fusion protein is generated by translation of a nucleic acid in which a polynucleotide encoding all or a portion of an IL11 protein is joined in-frame with a polynucleotide encoding all or a portion of albumin) to one another. The IL11 protein and albumin protein, once part of the albumin fusion protein, may be referred to as a "portion", "region" or "moiety" of the albumin fusion protein.

In one embodiment the albumin fusion protein comprises HA as the N-terminal portion, and an IL11 protein as the C-terminal portion. Alternatively, an albumin fusion protein comprising HA as the C-terminal portion, and an IL11 protein as the N-terminal portion may also be used.

Additionally, the albumin fusion proteins of the invention may include a linker peptide between the fused portions to provide greater physical separation between the moieties and thus maximize the accessibility of the IL11protein portion, for instance, for binding to its cognate receptor. The linker peptide may consist of amino acids such that it is flexible or more rigid.

Therefore, as described above, the albumin fusion proteins of the invention may have the following formula R2-R1; R1-R2; R2-R1-R2; R2-L-R1-L-R2: R1-L-R2; R2-L-R1; or R1-L-R2-L-R.1, wherein R1 is at least one IL11 protein, peptide or polypeptide sequence (including fragments thereof), L is a linker and R2 is a serum albumin sequence (including fragments thereof). Exemplary linkers include (GGGGS)_{N} (SEQ ID NO:8) or (GGGS)_{N} (SEQ ID NO:9) or (GGS)_{N}, wherein N is an integer greater than or equal to 1 and wherein G represents glycine and S represents serine.

In further embodiments, albumin fusion proteins of the invention comprising an IL11 protein have extended shelf-life or *in vivo* half-life or therapeutic activity compared to the shelf-life or *in vivo* half-life or therapeutic activity of the same IL11 protein when not fused to albumin. Shelf-life typically refers to the time period over which the therapeutic activity of a IL11 protein in solution or in some other storage formulation, is stable without undue loss of therapeutic activity. .

Albumin fusion proteins of the invention with "prolonged" or "extended" shelf-life exhibit greater therapeutic activity relative to a standard that has been subjected to the same storage and handling conditions. The standard may be the unfused full-length IL11 protein. As an example, an albumin fusion protein of the invention may retain greater than about 100% of the therapeutic activity, or greater than about 105%, 110%, 120%, 130%, 150% or 200% of the therapeutic activity of a standard when subjected to the same storage and handling conditions as the standard when compared at a given time point. However, it is noted that the therapeutic activity depends on the IL11 protein's stability, and may be below 100%.

Shelf-life may also be assessed in terms of therapeutic activity remaining after storage, normalized to therapeutic activity when storage began. Albumin fusion proteins of the invention with prolonged or extended shelf-life as exhibited by prolonged or extended therapeutic activity may retain greater than about 50% of the therapeutic activity, about 60%, 70%, 80%, or 90% or more of the therapeutic activity of the equivalent unfused IL11 protein when subjected to the same conditions.

IL11 proteins As stated above, an albumin fusion protein of the invention comprises at least a fragment of an IL11 protein and at least a fragment of human serum albumin, which are associated with one another by genetic fusion.

As used herein, "Therapeutic protein" refers to IL-11, or fragments thereof, having one or -more therapeutic and/or biological activities. Thus an albumin fusion protein of the invention may contain at least a fragment of an IL11 protein. Additionally, the term "therapeutic protein" may refer to the endogenous or naturally occurring correlate of an IL11 protein. include the endogenous or naturally occurring correlates of an IL11 protein.

By an IL11 polypeptide displaying a "therapeutic activity" or a protein that is "therapeutically active" is means an IL11 polypeptide that possesses one or more known biological and/or therapeutic activities associated with an IL11 protein such as one or more of the IL11 proteins described herein or otherwise known in the art. As a non-limiting example, an IL11 protein" is a protein that is useful to treat, prevent or ameliorate a disease, condition or disorder.

As used herein, "therapeutic activity" or "activity" may-refer to an activity whose effect is consistent with a desirable therapeutic outcome in humans, or to desired effects in non-human mammals or in other species or organisms. Therapeutic activity may be measured *in vivo* or *in vitro.* For example, a desirable effect may be assayed in cell culture. Such *in vitro* or cell culture assays are commonly available for many Therapeutic proteins as described in the art

Therapeutic proteins corresponding to a therapeutic protein portion of an albumin fusion protein of the invention may be modified by the attachment of one or more oligosaccharide groups. The modification, referred to as glycosylation, can dramatically affect the physical properties of proteins and can be important in protein stability, secretion, and localization. Such modifications are described in detail in WO 03/066824 and WO 01/79480, which are incorporated herein by reference

Therapeutic proteins corresponding to a Therapeutic protein portion of an albumin fusion protein of the invention, as well as analogs thereof, may be modified so that glycosylation at one or more sites is altered as a result of manipulation(s) of their nucleic acid sequence, by the host cell in which they are expressed, or due to other conditions of their expression. For example, glycosylation isomers may be produced by abolishing or introducing glycosylation sites, e.g., by substitution or deletion of amino acid residues, such as substitution of glutamine for asparagine, or unglycosylated recombinant proteins may be produced by expressing the proteins in host cells that will not glycosylate them, *e.g.* in *E*. *coli* or glycosylation-deficient yeast Examples of these approaches are described in more detail in WO 03/066824 and WO 01/79480, which are known in the art.

In various embodiments, the albumin fusion proteins of the invention are capable of a therapeutic activity and/or biologic activity corresponding to the therapeutic activity and/or biologic activity of the Therapeutic protein corresponding to the Therapeutic protein portion of the albumin fusion. In further embodiments, the therapeutically active protein portions of the albumin fusion proteins of the invention are fragments of the reference sequence and are capable of the therapeutic activity and/or biologic activity of the corresponding Therapeutic protein.

### Polypeptide and Polynucleotide Fragments and Variants

### Fragments

The present invention is further directed to fragments of the Therapeutic proteins, albumin proteins, and/or albumin fusion proteins of the invention.

Accordingly, fragments of a Therapeutic protein corresponding to a Therapeutic protein portion of an albumin fusion protein of the invention, include the full length protein as well as polypeptides having one or more residues deleted from the amino terminus of the amino acid sequence of the reference polypeptide. Polynucleotides encoding these polypeptides are also encompassed by the invention.

In addition, fragments of serum albumin polypeptides corresponding to an albumin protein portion of an albumin fusion protein of the invention, include the full length protein as well as polypeptides having one or more residues deleted from the amino terminus of the amino acid sequence of the reference polypeptide (i.e., serum albumin). Polynucleotides encoding these polypeptides are also encompassed by the invention.

Moreover, fragments of albumin fusion proteins of the invention include the full length albumin fusion protein as well as polypeptides having one or more residues deleted from the amino terminus of the albumin fusion protein. Polynucleotides encoding these polypeptides are also encompassed by the invention.

The present invention further provides polypeptides having one or more residues deleted from the carboxy terminus of the amino acid sequence of an IL11 protein corresponding to an IL11 protein portion of an albumin fusion protein of the invention. Polynucleotides encoding these polypeptides are also encompassed by the invention.

In addition, the present invention provides polypeptides having one or more residues deleted from the carboxy terminus of the amino acid sequence of an albumin protein corresponding to an albumin protein portion of an albumin fusion protein of the invention (e.g., serum albumin). Polynucleotides encoding these polypeptides are also encompassed by the invention.

Moreover, the present invention provides polypeptides having one or more residues deleted from the carboxy terminus of an albumin fusion protein of the invention. Polynucleotides encoding these polypeptides are also encompassed by the invention.

In addition, any of the above described N- or C-terminal deletions can be combined to produce a N- and C-terminal deleted reference polypeptide (e.g., a Therapeutic protein referred to in Table 1, or serum albumin (e.g., SEQ ID NO: 18), or an albumin fusion protein of the invention). The invention also provides polypeptides having one or more amino acids deleted from both the amino and the carboxyl termini. Polynucleotides encoding these polypeptides are also encompassed by the invention.

The present application is also directed to proteins containing polypeptides at least 60%, 80%, 85%, 90%, 95%, 96%, 97%, 98% or 99% identical to a reference polypeptide sequence (e.g., a Therapeutic protein, serum albumin protein or an albumin fusion protein of the invention) set forth herein, or fragments thereof. In some embodiments, the application is directed to proteins comprising polypeptides at least 60%, 80%, 85%, 90%, 95%, 96%, 97%, 98% or 99% identical to reference polypeptides having the amino acid sequence of N- and C-tenminal deletions as described above. Polynucleotides encoding these polypeptides are also encompassed by the invention.

Other polypeptide fragments of the invention are fragments comprising, or alternatively, consisting of, an amino acid sequence that displays a Therapeutic activity and/or functional activity (e.g. biological activity) of the polypeptide sequence of the IL11 or serum albumin protein of which the amino acid sequence is a fragment.

Other polypeptide fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

The polynucleotide variants of the invention may contain alterations in the coding regions, non-coding regions, or both. Polynucleotide variants include those containing alterations which produce silent substitutions, but do not alter the properties or activities of the encoded polypeptide. Such nucleotide variants may be produced by silent substitutions due to the degeneracy of the genetic code. Polypeptide variants include those in which less than 50, less than 40, less than 30, less than 20, less than 10, or 5-50, 5-25, 5-10, 1-5, or 1-2 amino acids are substituted, in any combination. Polynucleotide variants can be produced for a variety of reasons, e.g., to optimize codon expression for a particular host (change codons in the human mRNA to those preferred by a microbial host, such as, yeast or *E*. *coli*).

In another embodiment, a polynucleotide encoding an albumin portion of an albumin fusion protein of the invention is optimized for expression in yeast or mammalian cells. In a further embodiment, a polynucleotide encoding an IL 11 protein portion of an albumin fusion protein of the invention is optimized for expression in yeast or mammalian cells. In a still further embodiment, a polynucleotide encoding an albumin fusion protein of the invention is optimized for expression in yeast or mammalian cells.

Naturally occurring variants are called "allelic variants," and refer to one of several alternative forms of a gene occupying a given locus on a chromosome of an organism. (Genes II, Lewin, B., ed., John Wiley & Sons, New York (1985)). These allelic variants can vary at either the polynucleotide and/or polypeptide level and are included in the present invention. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids may be deleted from the N-terminus or C-terminus of the polypeptide of the present invention without substantial loss of biological function. See, e.g., Ron et al., J. Biol. Chem. 268: 2984-2988 (1993) (KGF variants) and Dobeli et al., J. Biotechnology 7:199-216 (1988) (interferon gamma variants).

Furthermore, even if deleting one or more amino acids from the N-terminus or C-terminus of a polypeptide results in modification or loss of one or more biological functions, other biological activities may still be retained.

In other embodiments, the variants of the invention have conservative substitutions. By "conservative substitutions" is intended swaps within groups such as replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Guidance concerning how to make phenotypically silent amino acid substitutions is provided, for example, in Bowie et al., "Deciphering the Message in Protein Sequences: Tolerance to Amino Acid Substitutions," Science 247:1306-1310 (1990), wherein the authors indicate that there are two main strategies for studying the tolerance of an amino acid sequence to change.

As the authors state, proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr_{;} replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly. Furthermore, chemical entities may be covalently attached to the albumin fusion proteins to enhance or modulate a specific functional or biological activity such as by methods disclosed in Current Opinions in Biotechnology, 10:324 (1999). Additional post-translational modifications encompassed by the invention include, for example, e.g., N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of prokaryotic host cell expression. The albumin fusion proteins may also be modified with, e.g., but not limited to, a chemotherapeutic agent, such as a drug, and/or a detectable label, such as an enzymatic, fluorescent, isotopic and/or affinity label to allow for detection and isolation of the protein. Examples of such modifications are given, e.g., in WO 03/066824 and in WO 01/79480 (pp. 105-106).

### Functional activity

"A polypeptide having functional activity" refers to an IL11 polypeptide capable of displaying one or more known functional activities associated with the full-length, pro-protein, and/or mature form of an IL11 protein. Such functional activities include, but are not limited to, biological activity, antigenicity (ability to bind (or compete with a polypeptide for binding) to an anti-polypeptide antibody), immunogenicity (ability to generate antibody which binds to a specific polypeptide of the invention), ability to form multimers with polypeptides of the invention, and ability to bind to a receptor or ligand for a polypeptide.

"A polypeptide having biological activity" refers to a polypeptide exhibiting activity similar to, but not necessarily identical to, an activity of an IL11 protein of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention.

In other embodiments, an albumin fusion protein of the invention has at least one biological and/or therapeutic activity associated with the IL11 protein (or fragment thereof) when it is not fused to albumin.

The albumin fusion proteins of the invention can be assayed for functional activity (e.g., biological activity) using or routinely modifying assays known in the art, as well as assays described herein. Specifically, albumin fusion proteins may be assayed for functioanal activity. Additionally, one of skill in the art may routinely assay fragments of an IL11 protein corresponding to an IL11 protein portion of an albumin fusion protein of the invention, for activity using assays for IL-11 activity. Further, one of skill in the art may routinely assay fragments of an albumin protein corresponding to an albumin protein portion of an albumin fusion protein of the invention, for activity using assays known in the art and/or as described in the Examples section in WO 03/066824 and WO 01/79480.

In addition, assays described herein (see Examples and Table 1) and otherwise known in the art may routinely be applied to measure the ability of albumin fusion proteins of the present invention and fragments, thereof to elicit biological activity and/or Therapeutic activity (either *in vitro* or *in vivo*) related to either the IL11 protein portion and/or albumin portion of the albumin fusion protein of the present invention. Other methods will be known to the skilled artisan and are within the scope of the invention.

### Expression of Fusion Proteins

The albumin fusion proteins of the invention may be produced as recombinant molecules by secretion from yeast, a microorganism such as a bacterium, or a human or animal cell line. Optionally, the polypeptide is secreted from the host cells.

For expression of the albumin fusion proteins exemplified herein, yeast strains disrupted in the *HSP150* gene as exemplified in WO 95/33833, or yeast strains disrupted in the *PMT1* gene as exemplified in WO 00/44772 (serving to reduce/eliminate O-linked glycosylation of the albumin fusions), or yeast strains disrupted in the *YAP3* gene as exemplified in WO 95/23857 were successfully used, in combination with the yeast *PRB1* promoter, the HSA/*MF*α-*1* fusion leader sequence exemplified in WO 90/01063, the yeast *ADH1* terminator, the *LEU2* selection marker and the disintegration vector pSAC35 exemplified in US 5,637,504.

Other yeast strains, promoters, leader sequences, terminators, markers and vectors which are expected to be useful in the invention are described in WO 03/066824 and in WO 01/74980 (pp. 94-99).

The present invention also includes a cell, optionally a yeast cell transformed to express an albumin fusion protein of the invention. In addition to the transformed host cells themselves, the present invention also contemplates a culture of those cells, optionally a monoclonal (clonally homogeneous) culture, or a culture derived from a monoclonal culture, in a nutrient medium. If the polypeptide is secreted, the medium will contain the polypeptide, with the cells, or without the cells if they have been filtered or centrifuged away. Many expression systems are know and may be used, including bacteria (for example *E*. *coli* and *Bacillus subtilis*), yeasts (for example *Saccharomyces cerevisiae, Kluyveromyces lactis* and *Pichia pastoris*), filamentous fungi (for example *Aspergillus*), plant cells, animal cells and insect cells.

The desired protein is produced in conventional ways, for example from a coding sequence inserted in the host chromosome or on a free plasmid. The yeasts are transformed with a coding sequence for the desired protein in any of the usual ways, for example electroporation. Methods for transformation of yeast by electroporation are disclosed in Becker & Guarente (1990) Methods Enzymol. 194, 182.

Successfully transformed cells, *i.e.*, cells that contain a DNA construct of the present invention, can be identified by well known techniques. For example, cells resulting from the introduction of an expression construct can be grown to produce the desired polypeptide. Cells can be harvested and lysed and their DNA content examined for the presence of the DNA using a method such as that described by Southern (1975) J. Mol. Biol. 98, 503 or Berent et al. (1985) Biotech. 3, 208. Alternatively, the presence of the protein in the supernatant can be detected using antibodies.

Useful yeast plasmid vectors include pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3.* Plasmids pRS413-416 are Yeast Centromere plasmids (YCps).

Vectors for making albumin fusion proteins for expression in yeast include pPPC0005, pScCHSA, pScNHSA, and pC4:HSA which were deposited on April 11, 2001 at the American Type Culture Collection, 10801 University Boulevard, Manassas, Virginia 20110-2209 and which are described in WO 03/066824 and WO 01/79480.

Another vector which is expected to be useful for expressing an albumin fusion protein in yeast is the pSAC35 vector which is described in Sleep et al., BioTechnology 8:42 (1990). The plasmid pSAC35 is of the disintegration class of vector described in US 5,637 504.

A variety of methods have been developed to operably link DNA to vectors via complementary cohesive termini. For instance, complementary homopolymer tracts can be added to the DNA segment to be inserted to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. The DNA segment, generated by endonuclease restriction digestion, is treated with bacteriophage T4 DNA polymerase *or E. coli* DNA polymerase I, which are enzymes that remove protruding, 5'-single-stranded termini with their 3' -5'-exonucleolytic activities and fill in recessed 3'-ends with their polymerizing activities. The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces terming compatible with those of the DNA segment.

Synthetic linkers containing a variety of restriction endonuclease sites are commercially available from a number of commercial sources.

Exemplary genera of yeast contemplated to be useful in the practice of the present invention as hosts for expressing the albumin fusion proteins are *Pichia* (formerly classified as Hansenula), *Saccharomyces, kluyveromyces, Aspergillus, Candida, Torulopsis, Torulaspora, Schizosaccharomyces, Citeromyces, Pachysolen, Zygosaccharomyces, Debaromyces, Trichoderma, Cephalosporium, Humicola, Mucor, Neurospora, Yarrowia, Metschunikowia, Rhodosporidium, Leucosporidium, Botryoascus, Sporidiobolus, Endomycopsis,* and the like. Genera include those selected from the group consisting of *Saccharomyces, Schizosaccharomyces, Kluyveromyces, Pichia* and *Torulaspora.* Examples of *Saccharomyces spp.* are *S cerevisiae, S. italicus and S. rouxii.* Examples of other species, and methods of transforming them, are described in WO 03/066824 and WO 01/79480 (pp. 97-98).

Methods for the transformation of *S*. *cerevisiae* are taught generally in EP 251 744, EP 258 067 and WO 90/01063. Suitable promoters for *S. cerevisiae* include those associated with the *PGKI* gene, *GAL1* or *GAL10* genes, *CYCI, PHO5, TRPI, ADHI, ADH2,* the genes for glyceraldehyde-3-phosphate dehydrogenase, hexokinase, pyruvate decarboxylase, phosphofructokinase, triose phosphate isomerase, phosphoglucose isomerase, glucokinase, alpha-mating factor pheromone (a mating factor pheromone), the *PRB1* promoter, the *GUT2* promoter, the *GPDI* promoter, and hybrid promoters involving hybrids of parts of 5' regulatory regions with parts of 5' regulatory regions of other promoters or with upstream activation sites (*e.g.* the promoter of EP-A-258 067).

Convenient regulatable promoters for use in *Schizosaccharomyces pombe* are the thiamine-repressible promoter from the nmt gene as described by Maundrell (1990) J. Biol. Chem. 265, 10857-10864 and the glucose repressible jbpl gene promoter as described by Hoffman & Winston (1990) Genetics 124, 807-816.

Methods of transforming *Pichia* for expression of foreign genes are taught in, for example, Cregg *et al.* (1993), and various Phillips patents (*e.g.* US 4 857 467, and *Pichia* expression kits are commercially available from Invitrogen BV, Leek, Netherlands, and Invitrogen Corp., San Diego, California. Suitable promoters include AOXI and AOX2. Gleeson et al. (1986) J. Gen. Microbiol. 132, 3459-3465 include information on *Haresenula* vectors and transformation, suitable promoters being MOX1 and FMD1; whilst EP 361 991, Fleer et al. (1991) and other publications from Rhone-Poulenc Rorer teach how to express foreign proteins in *Kluyveromyces* spp.

The transcription termination signal may be the 3' flanking sequence of a eukaryotic gene which contains proper signals for transcription termination and polyadenylation. Suitable 3' flanking sequences may, for example, be those of the gene naturally linked to the expression control sequence used, *i.e*. may correspond to the promoter. Alternatively, they may be different in which case the termination signal of the *S. cerevisiae ADHI* gene is optionally used.

The desired albumin fusion protein may be initially expressed with a secretion leader sequence, which may be any leader effective in the yeast chosen. Leaders useful in *S*. *cerevisiae* include that from the mating factor polypeptide (MFα1) and the hybrid leaders of EP-A-387 319. Such leaders (or signals) are cleaved by the yeast before the mature albumin is released into the surrounding medium. Further such leaders include those of *S*. *cerevisiae* invertase (SUC2) disclosed in JP 62-096086 (granted as 911036516), acid phosphatase (PH05), the pre-sequence of MFα-1, β-glucanase (BGL2) and killer toxin; *S*. *diastaticus* glucoamylase II; *S*. *carlsbergensis* α-galactosidase (MEL1); *K lactis* killer toxin; and *Candida glucoamylase.*

### Additional Methods of Recombinant and Synthetic Production of Albumin Fusion Proteins

The present invention includes polynucleotides encoding albumin fusion proteins of this invention, as well as vectors, host cells and organisms containing these polynucleotides. The present invention also includes methods of producing albumin fusion proteins of the invention by synthetic and recombinant techniques. The polynucleotides, vectors, host cells, and organisms may be isolated and purified by methods known in the art

A vector useful in the invention may be, for example, a phage, plasmid, cosmid, mini-chromosome, viral or retroviral vector. The vectors which can be utilized to clone and/or express polynucleotides of the invention are vectors which are capable of replicating and/or expressing the polynucleotides in the host cell in which the polynucleotides are desired to be replicated and/or expressed. In general, the polynucleotides and/or vectors can be utilized in any cell, either eukaryotic or prokaryotic, including mammalian cells (e.g., human (e.g., HeLa), monkey (e.g., Cos), rabbit (e.g., rabbit reticulocytes), rat, hamster (e.g., CHO, NSO and baby hamster kidney cells) or mouse cells (e.g., L cells), plant cells, yeast cells, insect cells or bacterial cells (e.g., *E. coli).* See, e.g., F. Ausubel et al., Current Protocols in Molecular Biology, Greene Publishing Associates and Wiley-Interscience (1992) and Sambrook *et al.* (1989) for examples of appropriate vectors for various types of host cells. Note, however, that when a retroviral vector that is replication defective is used, viral propagation generally will occur only in complementing host cells.

The host cells containing these polynucleotides can be used to express large amounts of the protein useful in, for example, pharmaceuticals, diagnostic reagents, vaccines and therapeutics. The protein may be isolated and purified by methods known in the art or described herein.

The polynucleotides encoding albumin fusion proteins of the invention may be joined to a vector containing a selectable marker for propagation in a host. Generally, a plasmid vector may be introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter compatible with the host cell in which the polynucleotide is to be expressed. The promoter may be a strong promoter and/or an inducible promoter. Examples of promoters include the phage lambda PL promoter, the *E. coli lac, trp, phoA* and *tac* promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs may include a translation initiating codon at the beginning and a termination codon (UAA, UGA or UAG) appropriately positioned at the end of the polypeptide to be translated.

As indicated, the expression vectors may include at least one selectable marker. Such markers include dihydrofolate reductase, G418, glutamine synthase, or neomycin resistance for eukaryotic cell culture, and tetracycline, kanamycin or ampicillin resistance genes for culturing in E. *coli* and other bacteria. Representative examples of appropriate hosts include, but are not limited to, bacterial cells, such as *E*. *coli, Streptomyces* and *Salmonella typhimurium* cells; fungal cells, such as yeast cells (e.g., *Saccharomyces cerevisiae* or *Pichia pastoris* (ATCC Accession No. 201178)); insect cells such as Drosophila S2 and Spodoptera Sf9 cells; animal cells such as CHO, COS, NSO, 293, and Bowes melanoma cells; and plant cells. Appropriate culture mediums and conditions for the above-described host cells are known in the art.

In one embodiment, polynucleotides encoding an albumin fusion protein of the invention may be fused to signal sequences which will direct the localization of a protein of the invention to particular compartments of a prokaryotic or eukaryotic cell and/or direct the secretion of a protein of the invention from a prokaryotic or eukaryotic cell. For example, in *E*. *coli,* one may wish to direct the expression of the protein to the periplasmic space. Examples of signal sequences or proteins (or fragments thereof) to which the albumin fusion proteins of the invention may be fused in order to direct the expression of the polypeptide to the periplasmic space of bacteria include, but are not limited to, the *pelB* signal sequence, the maltose binding protein (MBP) signal sequence, MBP, the *ompA* signal sequence, the signal sequence of the periplasmic *E*. *coli* heat-labile enterotoxin B-subunit, and the signal sequence of alkaline phosphatase. Several vectors are commercially available for the construction of fusion proteins which will direct the localization of a protein, such as the pMAL series of vectors (particularly the pMAL-p series) available from New England Biolabs. In a specific embodiment, polynucleotides albumin fusion proteins of the invention may be fused to *the pelB* pectate lyase signal sequence to increase the efficiency of expression and purification of such polypeptides in Gram-negative bacteria. *See,* U.S. Patent Nos. 5,576,195 and 5,846,818.

Examples of signal peptides that may be fused to an albumin fusion protein of the invention in order to direct its secretion in mammalian cells include, but are not limited to, the MPIF-1 signal sequence (e.g., amino acids 1-21 of GenBank Accession number AAB51134), the stanniocalcin signal sequence (MLQNSAVLLLLVISASA, SEQ ID NO:10) and a consensus signal sequence (MPTWAWWLFLVLLLALWAPARG, SEQ ID NO:11). A suitable signal sequence that may be used in conjunction with baculoviral expression systems is the gp67 signal sequence (e.g., amino acids 1-19 of GenBank Accession Number AAA72759).

Vectors which use glutamine synthase (GS) or DHFR as the selectable markers can be amplified in the presence of the drugs methionine sulphoximine or methotrexate, respectively. An advantage of glutamine synthase based vectors are the availability of cell lines (e.g., the murine myeloma cell line, NSO) which are glutamine synthase negative. Glutamine synthase expression systems can also function in glutamine synthase expressing cells (e.g., Chinese Hamster Ovary (CHO) cells) by providing additional inhibitor to prevent the functioning of the endogenous gene. A glutamine synthase expression system and components thereof are detailed in PCT publications: WO87/04462; WO86/05807; WO89/01036; WO89/10404; and WO91/06657. Additionally, glutamine synthase expression vectors can be obtained from Lonza Biologics, Inc. (Portsmouth, NH). Expression and production of monoclonal antibodies using a GS expression system in murine myeloma cells is described in Bebbington et al., Bio/technology 10:169(1992) and in Biblia & Robinson Biotechnol. Prog. 11:1 (1995).

The present invention also relates to host cells containing vector constructs, such as those described herein, and additionally encompasses host cells containing nucleotide sequences of the invention that are operably associated with one or more heterologous control regions (e.g., promoter and/or enhancer) using techniques known of in the art. The host cell can be a higher eukaryotic cell, such as a mammalian cell (e.g., a human derived cell), or a lower eukaryotic cell, such as a yeast cell, or the host cell can be a prokaryotic cell, such as a bacterial cell. A host strain may be chosen which modulates the expression of the inserted gene sequences, or modifies and processes the gene product in the specific fashion desired. Expression from certain promoters can be elevated in the presence of certain inducers; thus expression of the genetically engineered polypeptide may be controlled. Furthermore, different host cells have characteristics and specific mechanisms for the translational and post-translational processing and modification (e.g., phosphorylation, cleavage) of proteins. Appropriate cell lines can be chosen to ensure the desired modifications and processing of the foreign protein expressed.

Introduction of the nucleic acids and nucleic acid constructs of the invention into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

In addition to encompassing host cells containing the vector constructs discussed herein, the invention also encompasses primary, secondary, and immortalized host cells of vertebrate origin, particularly mammalian origin, that have been engineered to delete or replace endogenous genetic material (e.g., the coding sequence corresponding to an IL11 protein may be replaced with an albumin fusion protein corresponding to the IL11protein), and/or to include genetic material (e.g., heterologous polynucleotide sequences such as for example, an albumin fusion protein of the invention corresponding to the Therapeutic protein may be included). The genetic material operably associated with the endogenous polynucleotide may activate, alter, and/or amplify endogenous polynucleotides. In addition, techniques known in the art may be used to operably associate heterologous polynucleotides (e.g., polynucleotides encoding an albumin protein, or a fragment thereof) and/or heterologous control regions (e.g., promoter and/or enhancer) with endogenous polynucleotide sequences encoding an IL11 protein via homologous recombination (see, e.g., US 5,641,670; WO 96/29411; WO 94/12650; Koller et al., Proc. Natl. Acad Sci. USA 86:8932-8935 (1989); and Zijlstra et al., Nature 342:435-438 (1989).

Advantageously, albumin fusion proteins of the invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxyapatite chromatography, hydrophobic charge interaction chromatography and lectin chromatography. In some embodiments, high performance liquid chromatography ("HPLC") may be employed for purification.

In preferred some embodiments albumin fusion proteins of the invention are purified using one or more chromatography methods listed above. In other embodiments, albumin fusion proteins of the invention are purified using one or more of the following chromatography columns, Q Sepharose FF column, SP Sepharose FF column, Q Sepharose High Performance Column, Blue Sepharose FF column, Blue Column, Phenyl Sepharose FF column, DEAE Sepharose FF, or Methyl Column. "Sepharose" is a trademark.

Additionally, albumin fusion proteins of the invention may be purified using the process described in WO 00/44772. One of skill in the art could easily modify the process described therein for use in the purification of albumin fusion proteins of the invention.

Albumin fusion proteins of the present invention may be recovered from: products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, albumin fusion proteins of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

Albumin fusion proteins of the invention and antibodies that bind a Therapeutic protein or fragments thereof can be fused to marker sequences, such as a peptide to facilitate purification. In one embodiment, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad Sci. USA 86:821-824 (1989), for instance, hexa-histidine provides for convenient purification of the fusion protein. Other peptide tags useful for purification include, but are not limited to, the "HA" tag, which corresponds to an epitope derived from the influenza hemagglutinin protein (Wilson et al., Cell 37:767 (1954)) and the "FLAG" tag.

Further, an albumin fusion protein of the invention may be conjugated to a therapeutic moiety such as a cytotoxin, e.g., a cytostatic or cytocidal agent, a therapeutic agent or a radioactive metal ion, e.g., alpha-emitters such as, for example, 213Bi. Examples of such agents are given in WO 03/066824 and in WO 01/79480 (p. 107).

Albumin fusion proteins may also be attached to solid supports, which are particularly useful for immunoassays or purification of polypeptides that are bound by, that bind to, or associate with albumin fusion proteins of the invention. Such solid supports include, but are not limited to, glass, cellulose, polyacrylamide, nylon, polystyrene, polyvinyl chloride or polypropylene.

Also provided by the invention are chemically modified derivatives of the albumin fusion proteins of the invention which may provide additional advantages such as increased solubility, stability and circulating time of the polypeptide, or decreased immunogenicity (see U.S. Patent No. 4,179,337). Examples involving the use of polyethylene glycol are given in WO 01/79480 (pp. 109-111).

The presence and quantity of albumin fusion proteins of the invention may be determined using ELISA, a well known immunoassay known in the art.

### Uses of the Polypeptides

Each of the polypeptides identified herein can be used in numerous ways. The following description should be considered exemplary and utilizes known techniques.

The albumin fusion proteins of the present invention are useful for treatment, prevention and/or prognosis of various disorders in mammals, preferably humans. Such disorders include, but are not limited to thrombocytopenia, vWD and inflammatory diseases, such as IBD.

Moreover, albumin fusion proteins of the present invention can be used to treat or prevent diseases or conditions. In addition, the albumin fusion proteins of the invention may be used as a prophylactic measure Albumin fusion proteins can be used to assay levels of polypeptides in a biological sample. Albumin fusion proteins of the invention can also be used to raise antibodies, which in turn may be used to measure protein expression of the Therapeutic protein, albumin protein, and/or the albumin fusion protein of the invention from a recombinant cell, as a way of assessing transformation of the host cell, or in a biological sample. Moreover, the albumin fusion proteins of the present invention can be used to test the biological activities described herein.

### Transgenic Organisms

Transgenic organisms that express the albumin fusion proteins of the invention are also included in the invention. Transgenic organisms are genetically modified organisms into which recombinant, exogenous or cloned genetic material has been transferred. Such genetic material is often referred to as a transgene. The nucleic acid sequence of the transgene may include one or more transcriptional regulatory sequences and other nucleic acid sequences such as introns, that may be necessary for optimal expression and secretion of the encoded protein. The transgene may be designed to direct the expression of the encoded protein in a manner that facilitates its recovery from the organism or from a product produced by the organism, *e.g*. from the milk, blood, urine, eggs, hair or seeds of the organism. The transgene may consist of nucleic acid sequences derived from the genome of the same species or of a different species than the species of the target animal. The transgene may be integrated either at a locus of a genome where that particular nucleic acid sequence is not otherwise normally found or at the normal locus for the transgene.

The term "germ cell line transgenic organism" refers to a transgenic organism in which the genetic alteration or genetic information was introduced into a germ line cell, thereby conferring the ability of the transgenic organism to transfer the genetic information to offspring. If such offspring in fact possess some or all of that alteration or genetic information, then they too are transgenic organisms. The alteration or genetic information may be foreign to the species of organism to which the recipient belongs, foreign only to the particular individual recipient, or may be genetic information already possessed by the recipient. In the last case, the altered or introduced gene may be expressed differently than the native gene.

A transgenic organism may be a transgenic human, animal or plant. Transgenics can be produced by a variety of different methods including transfection, electroporation, microinjection, gene targeting in embryonic stem cells and recombinant viral and retroviral infection (*see, e.g.,* U.S. Patent No. 4,736,866; U.S. Patent No. 5,602,307; Mullins et al. (1993) Hypertension 22(4):630-633; Brenin et al. (1997) Surg. Oncol. 6(2)99-110; Tuan (ed.), Recombinant Gene Expression Protocols, Methods in Molecular Biology No. 62, Humana Press (1997)). The method of introduction of nucleic acid fragments into recombination competent mammalian cells can be by any method which favours co-transformation of multiple nucleic acid molecules. Detailed procedures for producing transgenic animals are readily available to one skilled in the art, including the disclosures in U.S. Patent No. 5,489,743 and U.S. Patent No. 5,602,307. Additional information is given in WO 03/066824 and WO 01/79480 (pp. 151-162).

### Gene Therapy

Constructs encoding albumin fusion proteins of the invention can be used as a part of a gene therapy protocol to deliver therapeutically effective doses of the albumin fusion protein. One approach for *in vivo* introduction of nucleic acid into a cell is by use of a viral vector containing nucleic acid, encoding an albumin fusion protein of the invention. Infection of cells with a viral vector has the advantage that a large proportion of the targeted cells can receive the nucleic acid. Additionally, molecules encoded within the viral vector, *e.g.*, by a cDNA contained in the viral vector, are expressed efficiently in cells which have taken up viral vector nucleic acid. The extended plasma half life of the described albumin fusion proteins might even compensate for a potentially low expression level. Retrovirus vectors and adeno-associated virus vectors can be used as a recombinant gene delivery system for the transfer of exogenous nucleic acid molecules encoding albumin fusion proteins *in vivo.* These vectors provide efficient delivery of nucleic acids into cells, and the transferred nucleic acids are stably integrated into the chromosomal DNA of the host. Examples of such vectors, methods of using them, and their advantages, as well as non-viral delivery methods are described in detail in WO 03/066824 and WO 01/79480 (pp. 151-153).

Gene delivery systems for a gene encoding an albumin fusion protein of the invention can be introduced into a patient by any of a number of methods. For instance, a pharmaceutical preparation of the gene delivery system can be introduced systemically, *e.g.* by intravenous injection, and specific transduction of the protein in the target cells occurs predominantly from specificity of transfection provided by the gene delivery vehicle, cell-type or tissue-type expression due to the transcriptional regulatory sequences controlling expression of the receptor gene, or a combination thereof. In other embodiments, initial delivery of the recombinant gene is more limited with introduction into the animal being quite localized. For example, the gene delivery vehicle can be introduced by catheter (see U.S. Patent 5,328,470) or by stereotactic injection (*e.g*. Chen et al. (1994) PNAS 91: 3054-3057). The pharmaceutical preparation of the gene therapy construct can consist essentially of the gene delivery system in an acceptable diluent, or can comprise a slow release matrix in which the gene delivery vehicle is imbedded. Where the albumin fusion protein can be produced intact from recombinant cells, *e.g.* retroviral vectors, the pharmaceutical preparation can comprise one or more cells which produce the albumin fusion protein. Additional gene therapy methods are described in WO 03/066824 and in WO 01/79480 (pp. 153-162).

### Pharmaceutical or Therapeutic Compositions

The albumin fusion proteins of the invention or formulations thereof may be administered by any conventional method including parenteral (*e.g.* subcutaneous or intramuscular) injection or intravenous infusion. The treatment may consist of a single dose or a plurality of doses over a period of times. Furthermore, the dose, or plurality of doses, is administered less frequently than for the Therapeutic Protein which is not fused to albumin.

While it is possible for an albumin fusion protein of the invention to be administered alone, it is desirable to present it as a pharmaceutical formulation, together with one or more acceptable carriers. The carrier(s) must be "acceptable" in the sense of being compatible with the albumin fusion protein and not deleterious to the recipients thereof. Typically, the carriers will be water or saline which will be sterile and pyrogen free. Albumin fusion proteins of the invention are particularly well suited to formulation in aqueous carriers such as sterile pyrogen free water, saline or other isotonic solutions because of their extended shelf-life in solution. For instance, pharmaceutical compositions of the invention may be formulated well in advance in aqueous form, for instance, weeks or months or longer time periods before being dispensed.

Formulations containing the albumin fusion protein may be prepared taking into account the extended shelf life of the albumin fusion protein in aqueous formulations. As discussed above, the shelf life of many of these Therapeutic proteins are markedly increased or prolonged after fusion to HA.

In instances where aerosol administration is appropriate, the albumin fusion proteins of the invention can be formulated as aerosols using standard procedures. The term "aerosol" includes any gas-borne suspended phase of an albumin fusion protein of the instant invention which is capable of being inhaled into the bronchioles or nasal passages. Specifically, aerosol includes a gas-bome suspension of droplets of an albumin fusion protein of the instant invention, as may be produced in a metered dose inhaler or nebulizer, or in a mist sprayer Aerosol also includes a dry powder composition of a compound of the instant invention suspended in air or other carrier gas, which may be delivered by insufflation from an inhaler device, for example.

The formulations may conveniently be presented in unit dosage form and may be prepared by any of the methods well known in the art of pharmacy. Such methods include the step of bringing into association the albumin fusion protein with the carrier that constitutes one or more accessory ingredients. In general the formulations are prepared by uniformly and intimately bringing into association the active ingredient with liquid carriers or finely divided solid carriers or both, and then, if necessary, shaping the product.

Formulations suitable for parenteral administration include aqueous and non-aqueous sterile injection solutions which may contain anti-oxidants, buffers, bacteriostats and solutes which render the formulation appropriate for the intended recipient; and aqueous and non-aqueous sterile suspensions which may include suspending agents and thickening agents. The formulations may be presented in unit-dose or multi-dose containers, for example sealed ampoules, vials or syringes, and may be stored in a freeze-dried (lyophilised) condition requiring only the addition of the sterile liquid carrier, for example water for injections, immediately prior to use. Extemporaneous injection solutions and suspensions may be prepared from sterile powders. Dosage formulations may contain the Therapeutic protein portion at a lower molar concentration or lower dosage compared to the non-fused standard formulation for the Therapeutic protein given the extended serum half-life exhibited by many of the albumin fusion proteins of the invention.

As an example, when an albumin fusion protein of the invention comprises one or more of the Therapeutic protein regions, the dosage form can be calculated on the basis of the potency of the albumin fusion protein relative to the potency of the Therapeutic protein, while taking into account the prolonged serum half-life and shelf-life of the albumin fusion proteins compared to that of the native Therapeutic protein. For example, in an albumin fusion protein consisting of a full length HA fused to a full length Therapeutic protein, an equivalent dose in terms of units would represent a greater weight of agent but the dosage frequency can be reduced.

Formulations or compositions of the invention may be packaged together with, or included in a kit with, instructions or a package insert referring to the extended shelf-life of the albumin fusion protein component. For instance, such instructions or package inserts may address recommended storage conditions, such as time, temperature and light, taking into account the extended or prolonged shelf-life of the albumin fusion proteins of the invention. Such instructions or package inserts may also address the particular advantages of the albumin fusion proteins of the inventions, such as the ease of storage for formulations that may require use in the field, outside of controlled hospital, clinic or office conditions. As described above, formulations of the invention may be in aqueous form and may be stored under less than ideal circumstances without significant loss of therapeutic activity.

The invention also provides methods of treatment and/or prevention of diseases or disorders (such as, for example, any one or more of the diseases or disorders disclosed herein) by administration to a subject of an effective amount of an albumin fusion protein of the invention or a polynucleotide encoding an albumin fusion protein of the invention ("albumin fusion polynucleotide") in a pharmaceutically acceptable carrier.

Effective dosages of the albumin fusion protein and/or polynucleotide of the invention to be administered may be determined through procedures well known to those in the art which address such parameters as biological half-life, bioavailability, and toxicity, including using data from routine in vitro and *in vivo* studies, using methods well known to those skilled in the art.

The albumin fusion protein and/or polynucleotide will be formulated and dosed in a fashion consistent with good medical practice, taking into account the clinical condition of the individual patient (especially the side effects of treatment with the albumin fusion protein and/or polynucleotide alone), the site of delivery, the method of administration, the scheduling of administration, and other factors known to practitioners. The "effective amount" for purposes herein is thus determined by such considerations.

For example, determining an effective amount of substance to be delivered can depend upon a number of factors including, for example, the chemical structure and biological activity of the substance, the age and weight of the patient, the precise condition requiring treatment and its severity, and the route of administration. The frequency of treatments depends upon a number of factors, such as the amount of albumin fusion protein or polynucleotide constructs administered per dose, as well as the health and history of the subject. The precise amount, number of doses, and timing of doses will be determined by the attending physician or veterinarian.

Albumin fusion proteins and polynucleotides of the present invention can be administered to any animal, preferably to mammals and birds. Preferred mammals include humans, dogs, cats, mice, rats, rabbits sheep, cattle, horses and pigs, with humans being particularly preferred.

As a general proposition, the albumin fusion protein of the invention will be dosed lower (on the molar basis of the unfused Therapeutic protein) or administered less frequently than the unfused Therapeutic protein. The albumin fusion proteins of the invention are advantageous in that they can simulate continuous infusion of "classic drugs", i.e., less protein equivalent is needed for identical inhibitory activity.

Albumin fusion proteins and/or polynucleotides can be are administered orally, rectally, parenterally, intracisternally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any. The term "parenteral" as used herein refers to modes of administration which include intravenous, intramuscular, intraperitoneal, intrasternal, subcutaneous and intra-articular injection and infusion.

Albumin fusion proteins and/or polynucleotides of the invention are also suitably administered by sustained-release systems such as those described in WO 03/066824 and WO 01/79480 (pp. 129-130).

For parenteral administration, in one embodiment, the albumin fusion protein and/or polynucleotide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation optionally does not include oxidizing agents and other compounds that are known to be deleterious to the Therapeutic.

The albumin fusion proteins and/or polynucleotides of the invention may be administered alone or in combination with other therapeutic agents. Albumin fusion protein and/or polynucleotide agents that may be administered in combination with the albumin fusion proteins and/or polynucleotides of the invention include, but are not limited to, antiretroviral agents like protease, reverse transcriptase, integrase and assembly inhibitors, chemotherapeutic agents, antibiotics, steroidal and non-steroidal anti-inflammatories, conventional immunotherapeutic agents, and/or therapeutic treatments as described, e.g., in WO 03/066824 and WO 01/79480 (pp. 132-151). Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.

Pharmaceutical compositions suitable for use in the present invention include compositions wherein the active ingredients are contained in an effective amount to achieve its intended purpose. In certain embodiments, albumin fusion proteins and/or polynucleotides of the invention are administered in combination with antiretroviral agents, nucleoside/nucleotide reverse transcriptase inhibitors (NRTIs), non-nucleoside reverse transcriptase inhibitors (NNRTIs), and/or protease inhibitors (PIs).

The invention also provides a pharmaceutical pack or kit comprising one or more containers filled with one or more of the ingredients of the pharmaceutical compositions comprising albumin fusion proteins of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

Having generally described the invention, the same will be more readily understood by reference to the following examples, which are provided by way of illustration and are not intended as limiting.

Without further description, it is believed that one of ordinary skill in the art can, using the preceding description and the following illustrative examples, make and utilize the alterations detected in the present invention and practice the claimed methods. The following working examples therefore, specifically point out certain embodiments of the present invention, and are not to be construed as limiting in any way the remainder of the disclosure.

### Example 1: Preparation of albumin-fused IL-11

The recombinant albumin expression vectors pAYE645 and pAYE646 have been described previously in WO 2004/009819. Plasmid pAYE645 contained the HSA/MFα-1 fusion leader sequence, as well as the yeast *PRB1* promoter and the yeast *ADH1* terminator providing appropriate transcription promoter and transcription terminator sequences, is described in WO 2004/009819. Plasmid pAYE645 was digested to completion with the restriction enzyme *Afl*II and partially digested with the restriction enzyme *Hind*III and the DNA fragment comprising the 3' end of the yeast PRB1 promoter and the albumin coding sequence was isolated. Plasmid pDB2241, described in patent application WO 00/44772, was digested with *Afl*II/*Hind*III and the DNA fragment comprising the 5' end of the yeast PRB1 promoter and the yeast *ADH1* terminator was isolated. The *Afl*II/*Hind*III DNA fragment from pAYE645 was then cloned into the *Afl*II/*Hind*III pDB2241 vector DNA fragment to create the plasmid pDB2302. Plasmid pDB2302 was digested to completion with *Pac*I/*Xho*I and the 6.19kb fragment isolated, the recessed ends were blunt ended with T4 DNA polymerase and dNTPs, and religated to generate plasmid pDB2465. Plasmid pDB2465 was linearised with *Cla*I, the recessed ends were blunt ended with T4 DNA polymerase and dNTPs, and religated to generate plasmid pDB2533. Plasmid pDB2533 was linearised with *Bln*I, the recessed ends were blunt ended with T4 DNA polymerase and dNTPs, and religated to generate plasmid pDB2534. Plasmid pDB2534 was digested to completion with *BmgB*I/*Bgl*II, the 6.96kb DNA fragment isolated and ligated to one of two double stranded oligonucleotide linkers, VC053/VC054 and VC057/VC058 to create plasmid pDB2540, or VC055/VC056 and VC057/VC058 to create plasmid pDB2541.

A sample of a human IL11 cDNA was serially diluted from 100ng.mL⁻¹ to 10pg.mL⁻¹ (in 10 fold increments). PCR primers CF59 (SEQ ID No: 7) and CF60 (SEQ ID No: 12) were designed to allow the IL11 cDNA to be cloned as an N-terminal albumin fusion into pDB2541 linearised with *Bgl*II and *Cla*I*,* whilst at the same time deleting the codon encoding the N-terminal proline from the IL11 coding region. The DNA sequence of each primer were as follows:

A master mix was prepared as follows: 2mM MgCl₂ PCR Buffer, 10µM PCR dNTP's, 0.2µM CF59, 0.2µM CF60, 2U FastStart *Taq*. DNA polymerase. 1µL of the IL11 cDNA (10pg, 100pg, 1ng, 10ng 100ng) was added to 49µL of reaction mix.. The total reaction volume was 50µL. Perkin-Elmer Thermal Cycler 9600 was programmed as follows: Denature at 95°C for 4 mins [HOLD], then [CYCLE] denature at 95°C for 30s, anneal for 30s at 45°C, extend at 72°C for 60s for 20 cycles, followed by a [HOLD] 72°C for 600 s and then [HOLD] 4°C. The products of the PCR amplification were analysed by gel electrophoresis and a band of expected size (0.59kb) was observed. The 0.59kb DNA fragment was isolated from the 1%(w/v) agarose TAE gel using Gene Clean III Kit (BIO101 Inc.).

The PCR DNA fragment was digested to completion with the restriction endonucleases *Bgl*II/*Cla*I and the 0.58kb fragment was ligated into the 6.15kb pDB2541 *Bgl*II/*Cla*I vector DNA fragment to create plasmid pDB2567.

Appropriate yeast vector sequences were provide by a "disintegration" plasmid pSAC35 generally disclosed in EP-A-286 424 and described by Sleep, D., et al. (1991) Bio/Technology 9, 183-187. The 3.53kb *NotI* N-terminal (des-pro)IL11-albumin expression cassette was isolated from pDB2567, purified and ligated into *NotI* digested pSAC35 which had been treated with calf intestinal phosphatase, creating plasmid pDB2569 contained the *Not*I expression cassette in the same orientation to the *LEU2* selection marker, and plasmid pDB2570 contained the *Not*I expression cassette in the opposite orientation to the *LEU2* selection marker.

PCR primers CF61 and CF62 were designed to allow the IL11 cDNA to be cloned as a C-terminal albumin fusion into pDB2243 linearised with *Bsu36*I and partially digested with *Hind*III, whilst at the same time adding two "TAA" translation stop codons at the 3' end of the IL11 open reading frame. Plasmid pDB2243, previously described in patent application WO 00/44772, which contained the yeast *PRB1* promoter and the yeast *ADH1* terminator provided appropriate transcription promoter and transcription terminator sequences. The DNA sequence of the CF61 (SEQ ID No: 13) and CF62 (SEQ ID No: 14) primers was as follows:

A master mix was prepared as follows: 2mM MgCl₂ PCR Buffer, 10µM PCR dNTP's, 0.2µM CF61, 0.2µM CF62, 2U FastStart *Taq.* DNA polymerase. 1µL of the IL11 cDNA (10pg, 100pg, Ing, 10ng, 100ng) was added to 49µL of reaction mix. The total reaction volume was 50µL. Perkin-Elmer Thermal Cycler 9600 was programmed as follows: Denature at 95°C for 4 mins [HOLD], then [CYCLE] denature at 95°C for 30s, anneal for 30s at 45°C, extend at 72°C for 60s for 20 cycles, followed by a [HOLD] 72°C for 600 s and then [HOLD] 4°C. The products of the PCR amplification were analysed by gel electrophoresis and a band of expected size (0.55kb) was observed. The 0.59kb DNA fragment was isolated from the 1%(w/v) agarose TAB gel using Gene Clean III Kit (BIO101 Inc.).

The PCR DNA fragment was digested to completion with the restriction endonucleases *Bsu36*I/*Hind*III and the 0.55kb fragment was ligated into the 6.19kb pDB2243 *Bsu36I,* partially digested with *Hind*III vector DNA fragment to create plasmid pDB2568.

Appropriate yeast vector sequences were provide by a "disintegration" plasmid pSAC35 generally disclosed in EP-A-286 424 and described by Sleep, D., et al. (1991) Bio/Technology 9, 183-187. The 3.53kb *NotI* C-terminal albumin-IL11 expression cassette was isolated from pDB2568, purified and ligated into *Not*I digested pSAC35 which had been treated with calf intestinal phosphatase, creating plasmid pDB2571 contained the *Not*I expression cassette in the same orientation to the *LEU2* selection marker, and plasmid pDB2572 container the *Not*I expression cassette in the opposite orientation to the *LEU2* selection marker. Yeast strains disclosed in WO 95/23857, WO 95/33833 and WO 94/04687 were transformed to leucine prototrophy as described in Sleep D., et al. (2001) Yeast 18, 403-421. The transformants were patched out onto Buffered Minimal Medium (BMM, described by Kerry-Williams, S.M. et al. (1998) Yeast 14, 161-169) and incubated at 30°C until grown sufficiently for further analysis.

DNA sequence of the N-terminal IL11-albumin fusion open reading frame forms SEQ ID No:15.

Amino acid sequence of the N-terminal IL11-albumin fusion protein is presented as SEQ ID No: 16.

Amino acid sequence of the mature N-terminal IL11-albumin fusion protein forms SEQ ID No: 17.

DNA sequence of the C-terminal albumin-IL11 fusion open reading frame is SEQ ID No: 18.

And the corresponding amino acid sequence of the C-terminal albumin-IL11 fusion protein is SEQ ID No: 19.

The amino acid sequence of the mature C-terminal albumin-IL11 fusion protein is SEQ ID No: 20.

### Example 2: Purification

### C-Terminal IL11 Purification

The C-Terminal IL11 fusion contained high levels of clipped (i.e. not full length) material. It was purified using the standard rHA SP-FF conditions as described in WO 00/44772 but in a negative mode whereby the fusion was in the flowthrough. The flowthrough was adjusted to pH 8 and 2.5mS.cm⁻¹ and loaded on a standard rHA DE-FF equilibrated in 15mM potassium tetraborate. This was operated in a negative mode. The conductivity of the DE-FF flowthrough was increased to 15mS.cm⁻¹ and the material purified using standard rHA DBA chromatography with an extra elution of 50mM octanoate in the equilibration buffer. The material was then concentrated and diafiltered against 300mM glycine, 10mM phosphate pH7.

### N-Terminal IL11 Purification (type A)

The N-Terminal IL11 contained some clipped material. It was purified using the standard rHA SP-FF conditions as described in WO 00/44772. The majority was in the flowthrough but sufficient bound for it to be necessary for it to be eluted using the standard elution buffer containing 200mM NaCl. The eluate was then adjusted to pH 8 and 2.5mS.cm⁻¹ and purified using standard rHA DE-FF equilibrated in 15mM potassium tetraborate. The DE-FF was eluted using the standard rHA elution buffer. The purified material was then concentrated and diafiltered against 300mM glycine, 10mM phosphate pH7.

### N-Terminal EL11 Purification (type B)

The N-terminal IL-11 fusion protein contained some clipped material. This material was separated using a S-Sepharose-FF column according to the standard rHA SP-FF conditions. Free rHA remained on the column. Then the fusion protein in the flowthrough was adsorbed to a monoclonal antibody Sepharose specific for albumin. The column was washed with high salt and eluted at pH 2.5. The eluate was adjusted to neutral pH, concentrated and diafiltered against 300mM glycine, 10mMNa-phosphate pH 7.0.

### Protein Characterisation after Purification

**Table 1: IL-11 albumin fusion characterisation**

| | **C-Terminal Fusion** | **N-Terminal Fusion (Type A)** | **N-Terminal Fusion (Type B)** |
|---|---|---|---|
| % Purity by SDS-PAGE and colloidal blue staining | 77 | 90 | 89 |
| ESMS indication of post-translational modifications | Theoretical mass 85565. Measured mass 68000-70000. | Theoretical mass 85468. Measured mass 85471. Good evidence for correct primary structure. | Theoretical mass 85468. Measured mass 85471. Good evidence for correct primary structure. |
| N-Terminal Sequence | N/A | Correct NT sequence for IL 11. | |
| Endotoxin (EU.mL⁻¹) | 73 | 23 | 130 |
| Fusion Concentration (mg.ML⁻¹) | 1.5 | 2.7 | 1 |

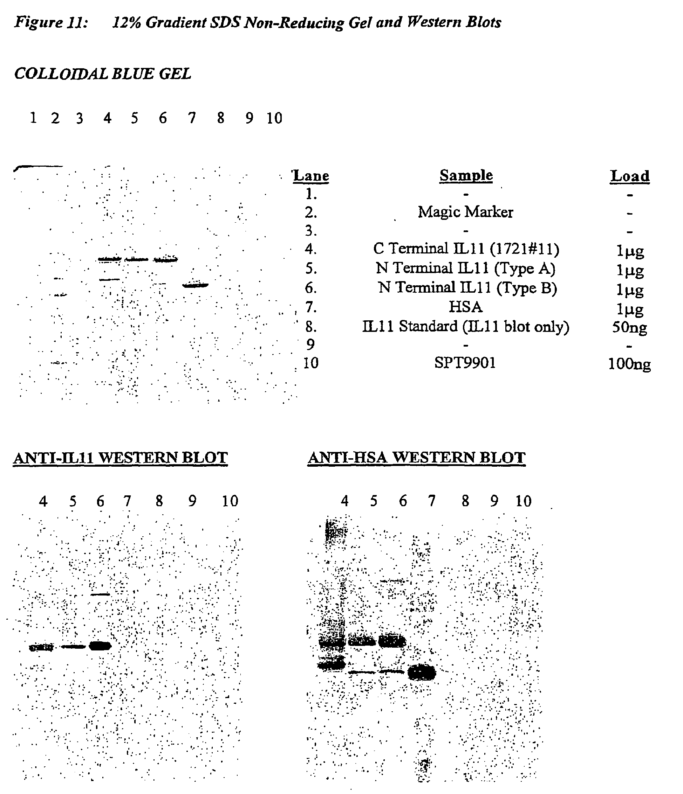

Images of the 12% Gradient SDS Non-Reducing Gel and Western Blots are shown in Figure 11.

### Example 3: Pharmacokinetics of albumin-fused IL-11 versus recombinant human IL-11 after single intravenous or subcutaneous administration to rabbits

Three male and three female rabbits per group received Neumega^{®} IL-11 (100 µg/kg) or C-terminal albumin-fused IL-11 (440 µg/kg) by a single i.v. or s.c. injection on day 0 (Table 2). Blood samples were drawn for the determination of the respective antigen levels at baseline and at 5 min, 10 min, 20 min, 30 min, 45 min, 1 h, 2 h, 4 h, 8 h, 24 h (1 d), 48 h (2 d), 72 h (3 d), 5 d, 7 d, 9 d, 11 d, and 14 d after i.v. administration of the respective test substance and at baseline, 30 min, 1 h, 2 h, 4 h, 8 h, 24 h (1 d), 48 h (2 d), 72 h (3 d), 5 d, 7 d, 9 d, 11 d and 14 d following s.c. injection. The doses of Neumega^{®} IL-11 and C-terminal albumin-fused IL-11 were calculated on an equimolar basis.

### Measurement of IL-11 plasma levels

Plasma levels of human IL-11 were measured by Quantikine^{®} Human IL-11 Immunoassay (R&D Systems, Catalog No. D1100). This assay employs the quantitative sandwich enzyme immunoassay technique. A murine monoclonal antibody specific to IL-11 has been coated onto a microplate. Standards and samples are pipetted into the wells and any IL-11 present is bound by the immobilized antibody. After washing away any unbound substances, an enzyme-linked polyclonal antibody specific for IL-11 is added to the wells. Following a wash to remove any unbound antibody-enzyme reagent, a substrate solution is added to the wells and colour develops in proportion to the amount of IL-11 bound in the initial step. The colour development is stopped and the intensity of the colour is measured.

### Plasma half life of fused vs. unfused protein in rabbits

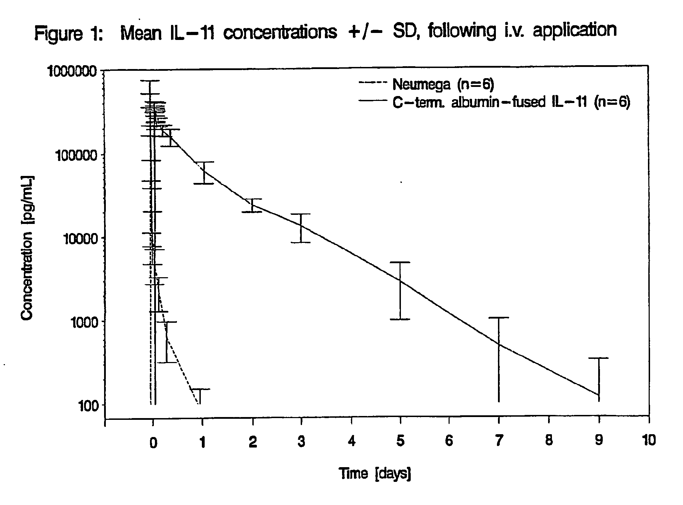

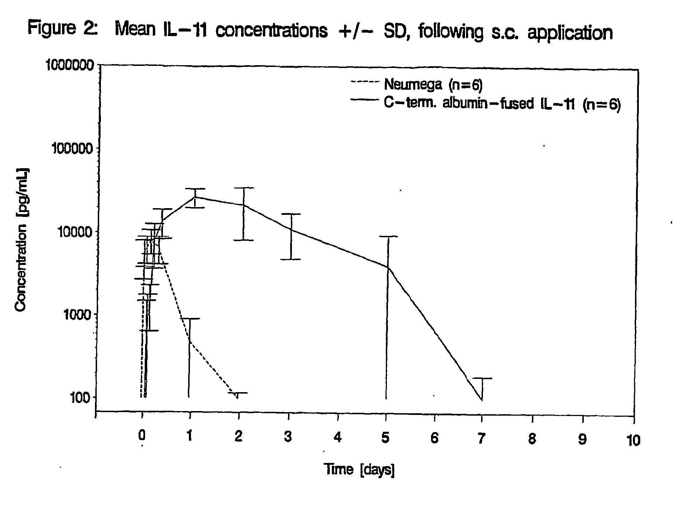

The means and standard deviations of the IL-11 concentrations at every time point are shown in Figure 1 for the i.v.-treated groups and in Figure 2 for the s.c. treated groups.

In the animals treated intravenously, levels above 100 pg/mL can be found for 1 day after injection in the Neumega^{®} IL-11 group and for 9 days after injection in the albumin-fused IL-11 group.

In the animals treated subcutaneously, the levels in the Neumega^{®} IL-11 group reach their peak around 6 hours after injection and stay above 100 pg/mL for little less than 2 days (Figure 2). The levels in the albumin-fused IL-11 group reach their peak 24 hours after injection and stay above 100 pg/mL for just under 7 days. The pharmacokinetic results are presented in Table 2 for the i.v.-treated groups and in Table 3 for the s.c.-treated groups.

**Table 2: Pharmacokinetic results following i.v. administration**

| | | **Neumega^{®} IL-11** | **C-terminal albumin-fused IL-11** |
|---|---|---|---|
| | N | 6 | 6 |
| **Initial half-life (hr)** | Mean | 0.12 | 3.61 |
| | Std Dev | 0.03 | 2.53 |
| | Median. | 0.11 | 3.32 |
| | Range | 0.09 - 0.15 | 0.30 - 6.52 |
| **Terminal half-life (hr)** | Mean | 3.31 | 22.79 |
| | Std Dev | 2.30 | 8.07 |
| | Median | 2.12 | 22.08 |
| | Range | 1.59 - 7.01 | 14.73 - 35.93 |
| **AUC₀₋₁₄ (hr·pg/mL)** | Mean | 162,686 | 5,421,764 |
| | Std Dev | 60,920 | 513,997 |
| | Median | 156,014 | 5,552,217 |
| | Range | 99,915 - 232,051 | 4,719,147 - 5,946,916 |
| | Geometric mean | 152,862 | 5,397,976 |
| | Scatter | 1.47 | 1.10 |
| | factor* | | |
| **Cₘₐₓ (pg/mL)** | Mean | 547,425 | 395,308 |
| | Std Dev | 194,193 | 61,216 |
| | Median | 540,825 | 379,975 |
| | Range | 274,200 - 760,450 | 332,300 - 489,100 |
| **Total clearance (mL/hr/kg)** | Mean | 721 | 21.0 |
| | Std Dev | 335 | 3.1 |
| | Median | 662 | 19.5 |
| | Range | 382 - 1,123 | 18.5 - 19.5 |
| **Total volume of distribution (mL/kg)** | Mean | 771 | 537 |
| | Std Dev | 636 | 170 |
| | Median | 628 | 500 |
| | Range | 204 - 1,781 | 377 - 847 |
| **Mean residence time (hr)** | Mean | 0.94 | 25.1 |
| | Std Dev | 0.49 | 4.3 |
| | Median | 0.81 | 25.2 |
| | Range | 0.44 - 1.63 | 20.4 - 31.9 |

| | | | |
|---|---|---|---|
| ** Scatter factor* = *exp[standard deviation (log-transformed values)]* | | | |

**Table 3: Pharmacokinetic results following s.c. administration**

| | | **Neumega^{®} IL-11** | **C-terminal albumin-fused IL-11** |
|---|---|---|---|
| | N | 6 | 6 |
| **Absorption half-life (hr)** | Mean | 1.14 | 16.5 |
| | Std Dev | 0.76 | 8.5 |
| | Median | 0.81 | 15.0 |
| | Range | 0.35 - 2.12 | 7.7 - 32.5 |
| **Terminal half-life (hr)** | Mean | 4.67 | 18.0 |
| | Std Dev | 1.43 | 3.9 |
| | Median | 4.40 | 19.2 |
| | Range | 3.07 - 6.67 | 11.7 - 22.3 |
| **AUC₀₋₁₄ (hr·pg/mL)** | Mean | 122,093 | 1,794,152 |
| | Std Dev | 28,159 | 753,505 |
| | Median | 118,860 | 1,665,227 |
| | Range | 95,376 - 172,688 | 1,113,203 - 3,000,848 |
| | Geometric mean | 119,635 | 1,670,725 |
| | Scatter factor* | 1.24 | 1.51 |
| **Cₘₐₓ (pg/mL)** | Mean | 8,098 | 30,590 |
| | Std Dev | 2,468 | 9,091 |
| | Median | 8,061 | 29,420 |
| | Range | 5,342 - 11,117 | 21,261 - 44,730 |
| **tₘₐₓ (hr)** Range | Mean | 5.67 | 28.0 |
| | Std Dev | 2.66 | 9.80 |
| | Median | 6.00 | 24.0 |
| | | 2.00 - 8.00 | 24.0 - 48_{.}0 |
| **Relative clearance (mL/hr/kg)** | **total** Mean | 1,270 | 70.9 |
| | Std Dev | 407 | 27.5 |
| | Median | 1,286 | 69.9 |
| | Range | 639 - 1,842 | 38.3 - 102.3 |
| **Relative volume of distribution (mL/kg)** | **total** Mean | 8,880 | 1753 |
| | Std Dev | 4,969 | 532 |
| | Median | 6,910 | 1,599 |
| | Range 4,512 - 15,836 | | 1,041 - 2,495 |

| | | | |
|---|---|---|---|
| ** Scatter factor* = *exp[standard deviation (log transformed values)]* | | | |

### Subcutaneous versus intravenous administration in rabbits

C-terminal albumin-fused IL-11 showed an average elimination half-life that was 8 times longer than that of Neumega^{®} IL-11 after i.v. application (Table 4). The area under the curve was 35 times larger. After s.c. injection, the average elimination half-life of C-terminal albumin-fused IL-11 was 4 times longer than that of Neumega^{®} IL-11. The area under the curve was 14 times larger.

**Table 4: Comparison of bioavailability between substances**

| **Rou te** | **Parameter** | **Elimination half-life** | **AUC₀₋₁₄** | **Cₘₐₓ** |
|---|---|---|---|---|
| **i.v.** | Estimated ratio (C-terminal albumin-fused IL-11/ Neumega^{®}) | 7.85 | 35.3 | 0.76 |
| | 90% confidence limits | 5.24 - 11.76 | 25.9 - 48.2 | 0.56 - 1.03 |
| **s.c.** | Estimated ratio (C-terminal albumin-fused IL-11/ Neumega^{®}) | 3.93 | 14.0 | 3.80 |
| | 90% confidence limits | 2.62 - 5.89 | 10.2 - 19.1 | 2.79 - 5.17 |

The bioavailability of Neumega^{®} s.c. versus i.v. was 78 % (Table 5) while for C-terminal albumin fusion it was calculated as 31 %.

**Table 5: Comparison of bioavailability between routes of application**

| **Substance** | **Parameter** | **Elimination half-life** | **AUC₀₋₁₄** | **Cₘₐₓ** |
|---|---|---|---|---|
| **Neumega^{®} IL-11** | Estimated ratio (s.c./i.v.) | 1.63 | 0.78 | 0.015 |
| | 90% confidence limits | 1.09 - 2.44 | 0.57 - 1.07 | 0.011 - 0.021 |
| **C-term. albumin-fused IL-11** | Estimated ratio (s.c. / i.v.) | 0.81 | 0.31 | 0.075 |
| | 90% confidence limits | 0.54 - 1.22 | 0.23 - 0.42 | 0.055 - 0.103 |

### Example 4: Pharmacokinetics of albumin-fused IL-11 versus recombinant human IL-11 after intravenous or subcutaneous administration to rats

Three rats per group received Neumega^{®} IL-11 (100 µg/kg) or N-terminal albumin-fused IL-11 (Type A) (440 µg/kg) by a single i.v. or s.c. injection on day 0 (Table 6). Blood samples for the determination of the respective antigen levels were drawn at the following timepoints: pre-injection, 2 min, 5 min, 10 min, 15 min, 30 min, 45 min, 1 h, 2 h, 8 h, 24 h, 48 h, 72 h (3d), 5 d, 7 d. The doses of Neumega^{®} IL-11 and N-terminal albumin-fused IL-11 were calculated on an equimolar basis.

### Study design

### Measurement of IL-11 plasma levels

Plasma levels for groups 1 and 3 (Neumega^{®} IL-11) were measured with an anti-human IL-11 ELISA by Quantikine^{®} Human IL-11 Immunoassay (R&D Systems, Catalog No. D1100), as described in Example 3 above. Plasma levels for groups 2 and 4 (IL-11-AFP) were determined with an anti-human albumin ELISA. The standard for this assay was IL-11-AFP.

### Plasma half life of fused vs. unfused protein in rats

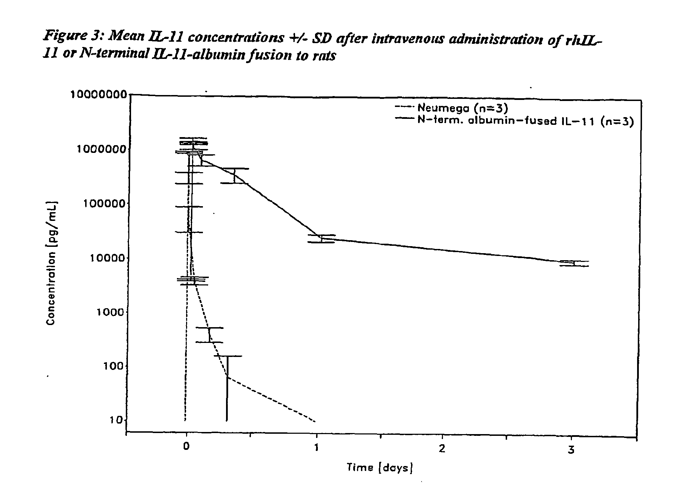

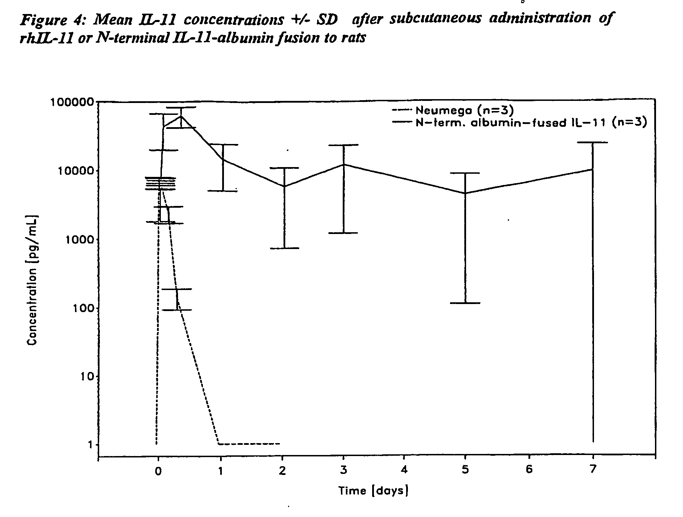

In the animals treated intravenously (Figure 3), levels above 0.1 ng/mL were found for 6 to 12 hours after injection in the Neumega^{®} group and for 3 days after injection in the albumin-fused IL-11 group. In the animals treated subcutaneously (Figure 4), the levels in the Neumega^{®} IL-11 group reached their peak within 1 hour after injection and stayed above 0.1 ng/mL for about 9 hours. The levels in the albumin-fused IL-11 group reached their peak between 2 and 8 hours after injection and stayed above 0.1 ng/mL for at least 44 hours.

The pharmacokinetic results are presented in Table 6 for the i.v. treated groups and in Table 7 for the s.c. treated groups.

**Table 6: Pharmacokinetic results following i.v. administration**

| | | **Neumega^{®} Il-11** | **N-terminal albumin-fused IL-11** |
|---|---|---|---|
| | N | 3 | 3 |
| **Initial life (hr)** | **half-** Mean | 0.13 | 3.15 |
| | Std Dev | 0.09 | 2.45 |
| | Median | 0.09 | 3.09 |
| | Range | 0.07 - 0.24 | 0.73 - 5.62 |
| **Terminal half-life (hr)** | Mean | 1.39 | 28.08 |
| | Std Dev | 0.92 | 23.10 |
| | Median | 0.92 | 24.57 |
| | Range | 0.80 - 2.46 | 6.93 - 52.73 |
| **AUC (hr·pg/mL)** | Mean | 26,375 | 8,357,724 |
| | Std Dev | 4,595 | 1,214,981 |
| | Median | 25,108 | 8,164,280 |
| | Range | 22,548 - 31,471 | 7,251,070 - 9,657,822 |
| | Geometric mean | 26,118 | 8,299,777 |
| | Scatter factor* | 1.19 | 1.15 |
| **Cₘₐₓ (pg/mL)** ** | Mean | 33,510 | 1,065,644 |
| | Std Dev | 4,310 | 133,261 |
| | Median | 31,565 | 1,134,432 |
| | Range | 30,51 - 38,450 | 912,045 - 1,150,455 |
| **Total clearance (mL/hr/kg)** | Mean | 4,324 | 14.1 |
| | Std Dev | 570 | 2.2 |
| | Median | 4,253 | 15.1 |
| | Range | 3,793 - 4,926 | 11.6 - 15.7 |
| **Total volume of distribution (mL/kg)** | Mean | 3,281 | 157 |
| | Std Dev | 538 | 27 |
| | Median | 2,971 | 150 |
| | Range | 2,970 - 3,902 | 134 - 187 |
| **Mean residence time (hr)** | Mean | 0.78 | 11.2 |
| | Std Dev | 0.22 | 2.1 |
| | Median | 0.70 | 11.9 |
| | Range | 0.60 - 1.03 | 8.9 - 12.9 |

| | | | |
|---|---|---|---|
| ** Scatter factor* = *exp[standard deviation (log-transformed values)]* *** measured at 10 minutes, the earliest post-injection Measurement in study PSR 04*/*02* | | | |

**Table 7: Pharmacokinetic results following s.c. administration**

| | | **Neumega^{®} Il-11** | **N-terminal albumin-fused IL-11** |
|---|---|---|---|
| | N | 3 | 3 |
| **Absorption half-life (hr)** | Mean | 0.74 | 2.63 |
| | Std Dev | 0.19 | 1.97 |
| | Median | 0.85 | 3.24 |
| | Range | 0.52 - 0.85 | 0.42 - 4.22 |
| **Terminal half-life (hr)** | Mean | 0.87 | 6.70 |
| | Std Dev | 0.04 | 4.84 |
| | Median | 0.86 | 4.66 |
| | Range | 0.85 - 0.92 | 3.21 - 12.23 |
| **AUC (hr·pg/mL)** | Mean | 24,687 | 1,032,955 |
| | Std Dev | 2,547 | 335,512 |
| | Median | 25,706 | 1,043,068 |
| | Range | 21,788 - 26,566 | 692,500 - 1,363,295 |
| | Geometric mean | 24,596 | 994,888 |
| | Scatter factor* | 1.11 | 1.41 |
| **Cₘₐₓ (pg/mL)** | Mean | 7,550 | 63,636 |
| | Std Dev | 135 | 14,205 |
| | Median | 7,612 | 62,159 |
| | Range | 7,395 - 7,642 | 50,227 - 78,523 |
| **tₘₐₓ (hr)** | Mean | 1.0 | 6.0 |
| | Std Dev | 0.0 | 3.5 |
| | Median | 1.0 | 8.0 |
| | Range | 1.0 - 1.0 | 2.0 - 8.0 |
| **Relative total clearance (mL/hr/kg)** | Mean | 4,168 | 112.0 |
| | Std Dev | 480 | 47.5 |
| | Median | 3,962 | 87.9 |
| | Range | 3,826 - 4,718 | 81.3 - 166.6 |
| **Relative total volume of distribution (mL/kg)** | Mean | 5,275 | 956 |
| | Std Dev | 842 | 527 |
| | Median | 4,858 | 771 |
| | Range | 4,723 - 6,244 | 547 - 1,550 |

| | | | |
|---|---|---|---|
| * *Scatter factor* = *exp[standard deviation (log-transformed values)]* | | | |

The maximum IL-11 concentrations measured in Study PSR 01/03 are presented in Table 8.

**Table 8: Maximum values obtained within one hour post-injection**

| | | **Neumega^{®} IL-11** | **N-terminal albumin-fused IL-11** |
|---|---|---|---|
| | | N 3 | 3 |
| **i.v. Cₘₐₓ** | Mean | 910,200 | 1,537,879 |
| **(pg/mL)** | Std Dev | 30,524 | 109,122 |
| | Median | 911,600 | 1,581,818 |
| | Range | 879,000 - 940,000 | 1,413,636 - 1,618,182 |
| **s.c. Cₘₐₓ** | Mean | 7,086 | 0 |
| **(pg/mL)** | Std Dev | 618 | 0 |
| | Median | 7,133 | 0 |
| | Range | 6,445 - 7,679 | 0 - 0 |
| **tₘₐₓ** | Mean | 0.42 | - |
| **(hours)** | Std Dev | 0.29 | - |
| | Median | 0.25 | - |
| | Range | 0.25 - 0.75 | 0.25 - 1.00 |

### Subcutaneous versus intravenous administration in rats

Table 9 shows the results of the analyses of variance regarding the relative bioavailability. The differences between the two products were significant with respect to elimination half-life, AUC and Cₘₐₓ for both routes of application.

**Table 9: Comparison of bioavailability between substances**

| **Route** | **Parameter half life** | **Elimination** | **AUC** | **Cₘₐₓ** |
|---|---|---|---|---|
| **i.v.** | Estimated ratio (N-terminal albumin-fused IL-11/ Neumega^{®}) | 17.03 | 317.8 | 1.69 |
| | 90% confidence limits | 5.98 - 48.49 | 230.8 - 437.5 | 1.41 - 2.02 |
| **s.c.** | Estimated ratio (N-terminal albumin-fused IL-11/ Neumega^{®}) | 6.50 | 40.5 | 8.29 |
| | 90% confidence limits | 2.28 - 18.50 | 29.4 - 55.7 | 6.92 - 9.93 |

Table 10 shows the results of the analyses of variance regarding the absolute bioavailability. For Neumega^{®} II-11, the differences between the two routes of application were not statistically significant with respect to elimination half-life and AUC. The difference in Cₘₐₓ was highly significant. For albumin-fused IL-11, the differences between the two routes of application were not significant with respect to elimination half-life. The differences regarding AUC and Cₘₐₓ were highly significant.

**Table 10: Comparison of bioavailability between routes of application**

| **Substance** | **Parameter** | **Elimination half-life** | **AUC** | **Cₘₐₓ^{a}** |
|---|---|---|---|---|
| **Neumega^{®} IL-11** | Estimated ratio (s.c. / i.v.) | 0.72 | 0.942 | 0.008 |
| | 90% confidence limits | 0.25 - 2.04 | 0.684 - 1.296 | 0.007 - 0.010 |
| **N-term. albumin-fused IL-11** | Estimated ratio (s.c. / i.v.) | 0.27 | 0.120 | 0.041 |
| | 90% confidence limits | 0.10 - 0.78 | 0.087 - 0.165 | 0.034 - 0.049 |

| | | | | |
|---|---|---|---|---|
| *^{a} Cₘₐₓ values of i.v. route taken from Study PSR 01*/*03, measured 2 minutes post-injection.* | | | | |

In rats N-terminal albumin-fused IL-11 showed an average elimination half-life that was 17 times longer than that of Neumega^{®} IL-11 after i.v. application. The area under the curve was 318 times larger. After s.c. injection, the average elimination half-life of N-terminal albumin-fused IL-11 was 6 times longer than that of Neumega^{®} IL-11. The area under the curve was 40 times larger.

### Example 5: Stimulation of thrombocytopoiesis in naive rats by IL-11-fusion protein

Naive CD-rats (10 per group) were treated with Neumega^{®} IL-11, C-terminal- or N-terminal-fused IL-11 (type B), or placebo according to the following schedule.

**Table 11: Treatment schedule**

| **Group** | **Substance** | **Dose** | **Appl. Sch.** | **Vol.** | **N** |
|---|---|---|---|---|---|
| **1** | Formulation solution | - | t= d 0 - d 9 s.c. | 0.5 ml | 10 |
| **2** | Neumega^{®} IL-11 | 100 µg /kg | t= d 0 - d 9 s.c. | 0.5 ml | 10 |
| **3** | IL-11-AFP C-term | 660 µg/kg | t= d 0, d 3, d 6, d 9 s.c. | 0.5 ml | 10 |
| **4** | IL-11-AFP N-term | 660 µg/kg | t= d 0, d 3, d 6, d 9 s.c. | 0.5 ml | 10 |
| **5** | IL-11-AFP C-term | 660 µg/kg | t= d 0 - d 9 s.c. | 0.5 ml | 10 |
| **6** | IL-11-AFP N-term | 660 µg/kg | t= d 0 - d 9 s.c. | 0.5 ml | 10 |

Blood samples were drawn at baseline, day 5, day 7, day 9, day 13 and day 16 and hematologic parameters (PLT, WBC, RBC, HCT, HGB) and body weight were measured. The dose of the fusion protein was calculated on an equimolar basis compared to Neumega^{®} IL-11 and corrected by a factor of 1.5 due to SDS-PAGE results.

### Results

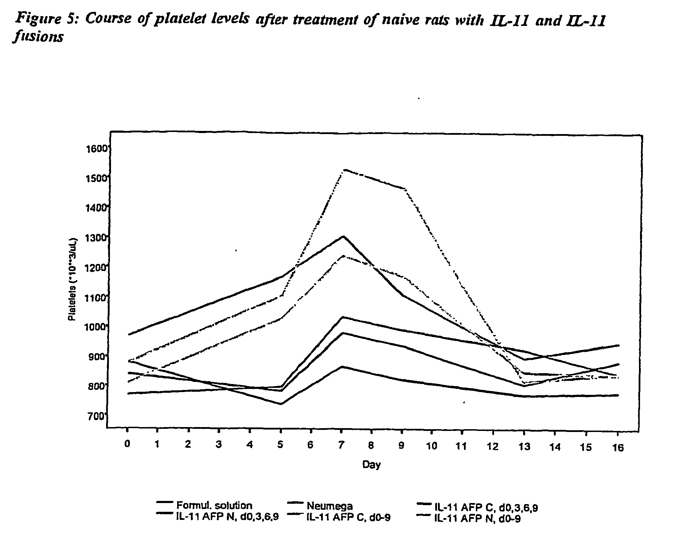

Maximum platelet counts were achieved in all groups on day 7 (Figure 5). The highest mean levels of 1528 x 103/µL were achieved with the N-terminal fusion. administered daily (group 6) or every three days (group 4) with 1304 x 103/µL. The C-terminal fusion also showed a dose-interval related effect with maximum mean levels of 1238 x 103/µL when administered daily compared to 977 x 103/µL if given every three days. Neumega^{®} IL-11 reached peak levels of 1032 x 103/µL while the control animals remained at 864 x 103/µL.

Red blood parameters (RBC, HGB, HCT) remained constant for all groups. White blood cell counts showed a slight initial increase followed by a non-consistent fluctuation. This can probably be attributed to the microtrauma caused by frequent injections and blood sampling. All animals showed a normal, slight increase in body weight over the course of the observation period.

In summary, both the N- and the C-terminal fusion administered daily as well as the N-terminal fusion administered in 3-day-intervals were clearly superior to control and Neumega^{®} IL-11 on days 5, 7 and 9. However, the N-terminal fusion seems to achieve higher levels than the C-terminal fusion.

The decrease of platelet counts after day 7 despite continuation of treatment until day 9 might occur due to the development of neutralizing antibodies to the heterologous human protein.

### Example 6: Treatment of chemotherapy-induced thrombocytopenia in rats by IL-11-fusion-protein

Ten female CD-rats per group received Carboplatin at 35 mg/kg i.v. on day 0. Starting on day 5 they were treated with Neumega^{®} IL-11, C-terminal- or N-terminal-fused IL-11 (type B), or placebo according to the following schedule.

**Table 12: Treatment schedule**

| **Group** | **Substance** | **Dose** | **Appl. Sch.** | **Vol.** | **N** |
|---|---|---|---|---|---|
| **1** | Formulation solution | - | t= d 5 - d 14 s.c. | 0.5 ml | 10 |
| **2** | Neumega^{®} | 50 µg/kg | t= d 5 - d 14 s.c. | 0.5 ml | 10 |
| **3** | IL-11-AFP C-term | 330 µg/kg | t= d 5, d 8, d 11, d 14 s.c. | 0.5 ml | 10 |
| **4** | IL-11-AFP N-term | 330 µg/kg | t= d 5, d 8, d 11, d 14 s.c. | 0.5 ml | 10 |
| **5** | IL-11-AFP C-term | 330 µg/kg | t= d 5 - d 14 s.c. | 0.5 ml | 10 |
| **6** | IL-11-AFP N-term | 330 µg/kg | t= d 5 - d 14 s.c. | 0.5 ml | 10 |

Blood samples were drawn at baseline, day 5, day 7, day 9, day 13 and day 16 and hematologic parameters (PLT, WBC, RBC, HCT, HGB) and body weight were measured. The dose of the fusion protein was calculated on an equimolar basis compared to Neumega^{®} IL-11 and corrected by a factor of 1.5 due to SDS-PAGE results.

### Results

In all groups platelet nadirs were observed between day 5 and day 9 (Table 13). Thereafter platelet levels recovered to above baseline on day 16. Mean platelet levels are shown in the following table.

**Table 13: Mean platelet levels (x 10³/µL)**

| | **Group 1** | **Group 2** | **Group 3** | **Group 4** | **Group 5** | **Group 6** |
|---|---|---|---|---|---|---|
| **day 0** | 782 | 783 | 788 | 703 | 813 | 810 |
| **day 5** | 477 | 281 | 368 | 458 | 344 | 365 |
| **day 7** | 388 | 302 | 303 | 330 | 348 | 374 |
| **day 9** | 365 | 323 | 365 | 375 | 328 | 432 |
| **day 13** | 603 | 702 | 681 | 832 | 689 | 1064 |
| **day 16** | 1003 | 1034 | 969 | 974 | 901 | 1287 |

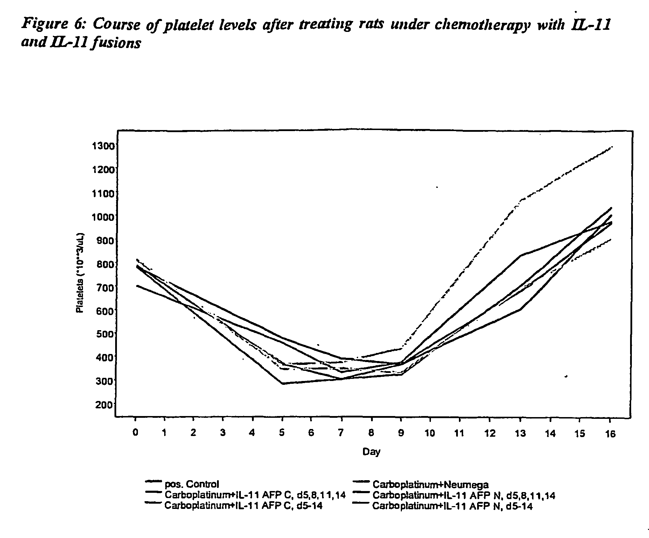

For Neumega^{®} IL-11, C- and N-terminal fusion injected daily, the platelet-nadir was observed on day 5, the first day of treatment (Figure 6). Thereafter in the group receiving the N-terminal fusion daily the platelet levels increased steadily and significantly, while the C-terminal fusion administered daily induced an increase comparable to Neumega^{®} IL-11.

An analysis of covariance adjusted for the day 5 platelet value shows a significantly higher platelet count for N-terminal fusion injected daily as compared to control on days 9, 13 and 16, as well as significant superiority over Neumega^{®} IL-11 on day 13. The same analysis demonstrates an overall treatment effect with p≤0.004 on days 7, 13 and 16.

Considering the duration of platelet levels below 500 x 10³/µL again the N-terminal fusion shows the best effects at a mean of 5.87 days when injected daily or 6.27 every 3 days as compared to 8.08 days for Neumega^{®} IL-11 and 6.93 days for controls. The C-terminal fusion shows less efficacy in that respect with a duration of 7.16 days with daily administration and 6.69 every 3 days.

Red blood parameters (RBC, HGB, HCT) showed a slight decrease during the study period in all groups, which might reflect the blood loss caused by frequent blood sampling. White blood cell counts showed a slight increase which can probably be attributed to the microtrauma caused by frequent injections and blood sampling. However this effect was especially prominent in the animals receiving N-terminal fusion of IL-11 daily, so that in this group a treatment related effect on WBC levels can not be excluded. All animals showed a normal, slight increase in body weight over the course of the observation period.

In summary the most prominent effect can be observed with the N-terminal albumin fusion of IL-11, while the C-terminal fusion behaves comparable to Neumega^{®} IL-11. Even though the absolute levels at nadir are not affected significantly, the N-terminal fusion protein is able to reduce the duration of levels below 500 x 10³/µL and leads to a significantly steeper recovery as compared to Neumega^{®} IL-11.

### Example 7: IL-11-fusion protein in a mouse model of inflammatory bowel disease (IBD)

In this model colitis is induced in mice through oral administration of 3 % dextran sulfate disodium salt (DSS) in tap water. Ten mice per group were allocated to the following treatment groups.

**Table 14: Treatment schedule**

| **Group** | **Treatment** | **Dose** | **Appl scheme** | **Volume** | **n** |
|---|---|---|---|---|---|
| **1** | Formulation solution, no DSS | n.a. | t = d 0-9 s.c.; 1x/day | 0.2 mL | 10 |
| **2** | Formulation solution | n.a. | t = d 0-9 s.c.; 1x/day | 0.2 mL | 10 |
| **3** | Sulfasalazine | 100 mg/kg | t = d 0-10; p.o. | ad lib. | 10 |
| **4** | Neumega^{®} IL-11 | 250 µg/kg | t = d 0-9 s.c.; 1x/day | 0.2 mL | 10 |
| **5** | IL-11-AFP C-term | 1650 µg/kg | t = d 0, 3, 6, 9 s.c.; 1x/day | 0.2 mL | 10 |
| **6** | IL-11-AFP N-term (type B) | 1650 µg/kg | t = d 0, 3, 6, 9 s.c.; 1x/day | 0.2 mL | 10 |

Body weight, rectal bleeding/diarrhoea and occult blood in faeces were assessed before induction and on days 3, 6 and 9. The experiment was terminated on day 10, the animals were necropsied, colon length was measured and samples of large intestine were retained for histological evaluation.

### Results

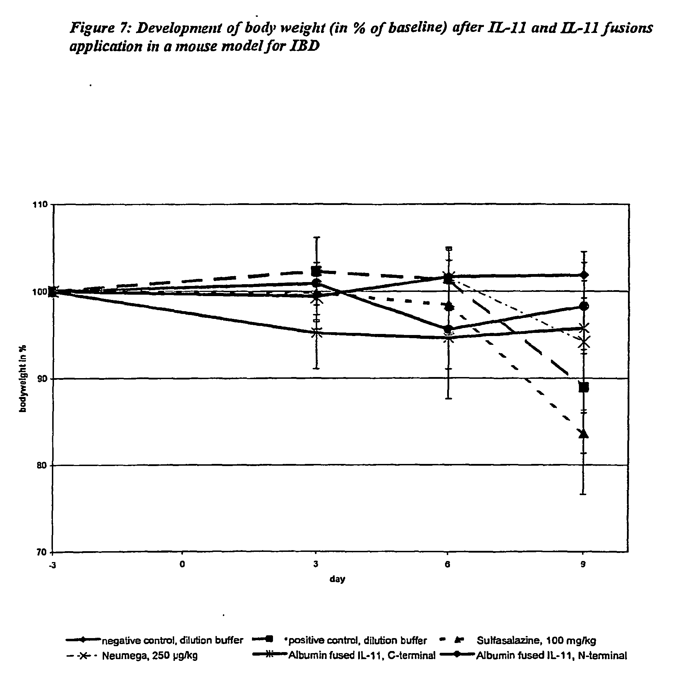

Animals receiving placebo, Sulfasalazine or Neumega^{®} IL-11 lost weight after the onset of colitis, while the animals treated with N- or C-terminal fusion even gained weight (Figure 7). This was a surprising effect that could not have been predicted in advance.

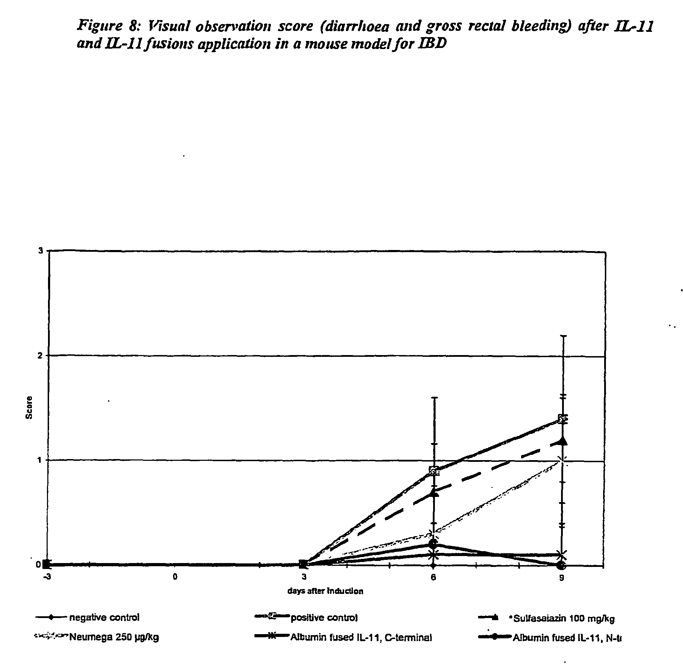

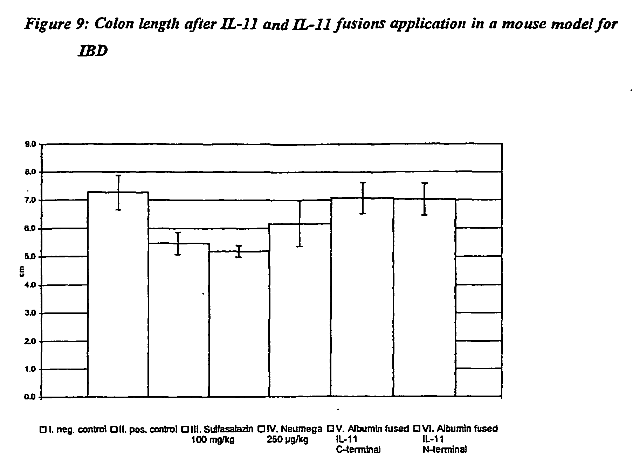

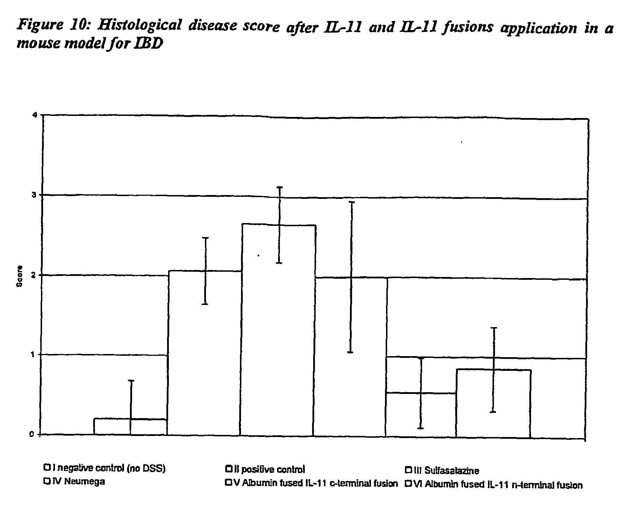

The visual observation score evaluating diarrhoea and rectal bleeding also shows that there was a surprising, and significant, beneficial effect of the fusion proteins compared to that of placebo, Sulfasalazine or Neumega^{®} IL-11 (Figure 8). The same holds true for the length of the colon (Figure 9) and the histological score (Figure 10).

In summary these data show that both albumin fusions given in 3 day intervals are able to significantly ameliorate the symptoms of DSS-induced colitis in mice, even superior to Neumega^{®} IL-11 or the current standard treatment Sulfasalazine.

### Example 8: Shelf-life

Shelf-life of the albumin-fused IL-11 products was determined according to the following method. A total of 25mg of the albumin-fused IL-11 products was formulated to a final volume of 5ml in a glass vial in sterile water-for-injection, with glycine, heptahydrate di-basic sodium phosphate, and mono-basic basic sodium phosphate as excipients Vials of the products were incubated at 25°C for 1 month. Samples were assayed at the end of one month as described in Example 1 for post-translational modifications, by ESMS, N-terminal sequence, non-reducing SDS polyacrylamide gels and Western blots. Shelf-life is considered to be prolonged if the compound exhibits fewer changes, in any one of these tests, after this storage than the unfused IL-11 compound.

### Example 9: Human Administration

At least in the case of the treatment of cancers, the albumin-fused IL-11 products may be administered by single s.c. injection once every four days, with a dose regime of 25-1000 µg per kg body weight, preferably 25-50 µg per kg.

### References

The present invention is not to be limited in scope by the specific embodiments described which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

## Claims

1. An albumin fusion protein comprising
i) full length human IL-11 or fragments of full length human IL-11 and
ii) full length human albumin or fragments of full length human albumin,
said fragments having one or more residues deleted from the amino terminus and/or the carboxy terminus wherein said fragments are at least 90% identical to full length human IL-11 and/or full length human albumin and wherein said deletions do not cause a substantial loss of biological function wherein the biological function is the promotion of thrombocytopoiesis.

2. The albumin fusion protein of claim 1 wherein the human IL-11 is fused to the N-terminus of human albumin.

3. The protein of claim 2, whereby the *in vivo* half-life of said albumin-fused IL-11 is extended at least 10-fold when administered intravenously in rats over the half-life of said IL-11 lacking the linked human albumin when also administered intravenously in rats.

4. The albumin fusion protein of claim 1 wherein the human IL-11 is fused to the C-terminus of the human albumin.

5. The albumin fusion protein of any preceding claim wherein the human IL-11 is separated from the human albumin by a linker.

6. The albumin fusion protein of any preceding claim wherein the *in vivo* biological activity of the human IL-11 fused to the human albumin is equal to or greater than the *in vivo* biological activity of said human IL-11 in an unfused state.

7. The albumin fusion protein of claim 6 wherein the biological activity is determined by the increase in mean platelet levels in a rat model of chemotherapy induced thrombocytopenia.

8. The albumin fusion protein of claim 6 wherein the biological activity is determined by the reduction of diarrhoea and rectal bleeding in a dextran sulphate disodium salt induced mouse model of inflammatory bowel disease.

9. A nucleic acid molecule comprising a polynucleotide sequence encoding the albumin fusion protein of any preceding claim.

10. A method for manufacturing an albumin fusion protein of any of claims 1 to 8, the method comprising (a) providing a nucleic acid comprising a nucleotide sequence encoding the albumin fusion protein expressible in a cell or organism; (b) expressing the nucleic acid in the cell or organism to form an albumin fusion protein; and (c) purifying the albumin fusion protein.

11. A pharmaceutical composition comprising an effective amount of the albumin fusion protein of any one of claims 1 to 8 and a pharmaceutically acceptable carrier or excipient.

12. An albumin fusion protein of any of claims 1 to 8 for use in medicine.

13. An albumin fusion protein of any of claims 1 to 8 for use in the treatment of thrombocytopenia and/or chemotherapy induced thrombocytopenia and/or inflammatory bowel disease and/or von Willebrand Disease.

14. An albumin fusion protein of any of claims 1 to 8 for use in minimizing a side effect associated with the treatment of a mammal with human IL-11 comprising administering said albumin fusion protein to said mammal.

15. An albumin fusion protein of any of claims 1 to 8 for use in minimizing weight loss, rectal bleeding or diarrhoea associated with the treatment of inflammatory bowel disease in a mammal with human IL-11 comprising administering said albumin fusion protein to said mammal

## Patentansprüche

1. Ein Albuminfusionsprotein enthaltend
i. vollständiges humanes IL-11 oder Fragmente von vollständigem humanem IL-11
und
ii. vollständiges humanes Albumin oder Fragmente von vollständigem humanem Albumin,
wobei besagte Fragmente Deletionen von einem oder mehreren Aminosäureresten am Aminoterminus und/oder am Carboxyterminus aufweisen, wobei besagte Fragmente mindestens 90% identisch zu vollständigem humanem IL-11 und/oder vollständigem humanem Albumin sind und wobei besagte Deletionen nicht einen wesentlichen Verlust biologischer Funktion bewirken, wobei die biologische Funktion die Förderung der Thrombozytopoese ist.

2. Albuminfusionsprotein nach Anspruch 1, wobei das humane IL-11 mit dem N-Terminus von humanem Albumin fusioniert ist.

3. Das Protein nach Anspruch 2, wobei die *in vivo* Halbwertszeit von besagtem mit Albumin fusionierten IL-11 nach intravenöser Applikation in Ratten mindestens 10fach verlängert ist verglichen mit der Halbwertszeit von IL-11 ohne verbundenes humanes Albumin welches ebenfalls intravenös in Ratten appliziert wird.

4. Das Albuminfusionsprotein nach Anspruch 1, wobei das humane IL-11 an den C-Terminus von humanem Albumin fusioniert ist.

5. Albuminfusionsprotein nach einem der vorgehenden Ansprüche, wobei das humane IL-11 von dem humanem Albumin durch einen Linker getrennt ist.

6. Albuminfusionsprotein nach einem der vorhergehenden Ansprüche, wobei die *in vivo* biologische Aktivität des an humanes Albumin fusionierten humanem IL-11 gleich oder größer ist, als die *in vivo* biologische Aktivität von besagtem humanem IL-11 welches nicht fusioniert ist.

7. Das Albuminfusionsprotein nach Anspruch 6 wobei die biologische Aktivität durch Anstieg des mittleren Plättchenspiegels in einem Rattenmodell von durch Chemotherapie induzierter Thrombozytopenie bestimmt wird.

8. Das Albuminfusionsprotein nach Anspruch 6 wobei die biologische Aktivität durch die Verminderung von Diarrhöe und rektalen Blutungen in einem durch Dextransulfat-Dinatrium-Salz induziertem Mausmodell von entzündlicher Darmerkrankung bestimmt wird.

9. Eine Nukleinsäure welche ein Polynukleotid beinhaltet, welches das Albuminfusionsprotein nach einem der vorgehenden Ansprüche kodiert.

10. Eine Herstellungsverfahren für ein Albuminfusionsprotein nach einem der Ansprüche 1 bis 8, wobei die Methode umfasst (a) zur Verfügung Stellung einer Nukleinsäure welche eine Nukleotidsequenz enthält, welche für ein Albuminfusionsprotein kodiert, welches in einer Zelle oder Organismus exprimierbar ist; (b) die Expression der Nukleinsäure in der Zelle oder dem Organismus unter Bildung eines Albuminfusionsproteins; und (c) Reinigung des Albuminfusionsproteins.

11. Eine pharmazeutische Zusammensetzung welche eine wirksame Menge eines Albuminfusionsproteins nach einem der Ansprüche 1 bis 8 und eine pharmazeutisch akzeptable Trägersubstanz oder Vehikel enthält.

12. Ein Albuminfusionsprotein nach einem der Ansprüche 1 bis 8 zur medizinischen Verwendung.

13. Ein Albuminfusionsprotein nach einem der Ansprüche 1 bis 8 zur Behandlung einer Thrombozytopenie, und/oder einer durch Chemotherapie induzierten Thrombozytopenie und/oder zur Behandlung einer entzündlichen Darmkrankheit und/oder zur Behandlung der von Willebrand Krankheit.

14. Ein Albuminfusionsprotein nach einem der Ansprüche 1 bis 8 zur Minimierung von Nebenwirkungen welche mit der Behandlung eines Säuger durch IL-11 assoziiert sind, welche die Applikation von besagtem Albuminfusionsprotein in besagtem Säuger beinhaltet.

15. Ein Albuminfusionsprotein nach einem der Ansprüche 1 bis 8 zur Minimierung von Gewichtsverlust, rektalen Blutungen und Diarrhöe welche mit der Behandlung von entzündlicher Darmerkrankung eines Säuger durch IL-11 assoziiert sind, welche die Applikation von besagtem Albuminfusionsprotein in besagtem Säuger beinhaltet.

## Revendications

1. Protéine de fusion de l'albumine, comprenant
i) de l'IL-11 humaine complète ou des fragments de l'IL-11 humaine complète, et
ii) de l'albumine humaine complète ou des fragments de l'albumine humaine complète,
lesdits fragments ayant un ou plusieurs résidus délétés du site amino-terminal et/ou du site carboxy-terminal, lesdits fragments ayant une identité d'au moins 90 % avec l'IL-11 humaine complète et/ou l'albumine humaine complète, et lesdites délétions ne provoquant pas une perte sensible de fonction biologique, la fonction biologique étant la promotion de la thrombocytopoïèse.

2. Protéine de fusion de l'albumine de la revendication 1, dans laquelle l'IL-11 humaine est fusionnée au site N-terminal de l'albumine humaine.

3. Protéine de la revendication 2, pour laquelle la demi-vie *in vivo* de ladite IL-11 fusionnée à l'albumine est multipliée par au moins 10, après administration par voie intraveineuse à des rats, par rapport à la demi-vie de ladite IL-11 ne contenant pas l'albumine humaine liée, elle aussi administrée par voie intraveineuse à des rats.

4. Protéine de fusion de l'albumine de la revendication 1, dans laquelle l'IL-11 humaine est fusionnée au site C-terminal de l'albumine humaine.

5. Protéine de fusion de l'albumine de l'une quelconque des revendications précédentes, dans laquelle l'IL-11 humaine est séparée de l'albumine humaine par un lieur.

6. Protéine de fusion de l'albumine de l'une quelconque des revendications précédentes, dans laquelle l'activité biologique *in vivo* de l'IL-11 humaine fusionnée à l'albumine humaine est supérieure ou égale à l'activité biologique *in vivo* de ladite IL-11 humaine dans un état non fusionné.

7. Protéine de fusion de l'albumine de la revendication 6, dans laquelle l'activité biologique est déterminée par une augmentation du niveau moyen des plaquettes dans un modèle rat de thrombocytopénie induite par une chimiothérapie.

8. Protéine de fusion de l'albumine de la revendication 6, dans laquelle l'activité biologique est déterminée par la réduction de la diarrhée et de l'hémorragie rectale dans un modèle souris, induit par le sel disodique du sulfate de dextran, de l'affection abdominale inflammatoire.

9. Molécule d'acide nucléique comprenant une séquence polynucléotidique codant pour la protéine de fusion de l'albumine de l'une quelconque des revendications précédentes.

10. Procédé de fabrication d'une protéine de fusion de l'albumine de l'une quelconque des revendications 1 à 8, le procédé comprenant (a) la mise à disposition d'un acide nucléique comprenant une séquence nucléotidique codant pour la protéine de fusion de l'albumine pouvant être exprimée dans une cellule ou un organisme ; (b) l'expression de l'acide nucléique dans la cellule ou dans l'organisme pour former une protéine de fusion de l'albumine ; et (c) la purification de la protéine de fusion de l'albumine.

11. Composition pharmaceutique comprenant une quantité efficace de la protéine de fusion de l'albumine de l'une quelconque des revendications 1 à 8 et un support ou un excipient pharmaceutiquement acceptable.

12. Protéine de fusion de l'albumine de l'une quelconque des revendications 1 à 8, pour utilisation en médecine.

13. Protéine de fusion de l'albumine de l'une quelconque des revendications 1 à 8, pour utilisation dans le traitement de la thrombocytopénie et/ou pour utilisation dans le traitement de la thrombocytopénie provoquée par une chimiothérapie et/ou pour utilisation dans le traitement de l'affection abdominale inflammatoire et/ou pour utilisation dans le traitement de la maladie de von Willebrand.

14. Protéine de fusion de l'albumine de l'une quelconque des revendications 1 à 8, pour utilisation dans le but de minimiser un effet secondaire associé au traitement d'un mammifère par de l'IL-11 humaine, comprenant l'administration de ladite protéine de fusion de l'albumine audit mammifère.

15. Protéine de fusion de l'albumine de l'une quelconque des revendications 1 à 8, pour utilisation dans le but de minimiser la perte de poids, l'hémorragie rectale ou la diarrhée associées au traitement de l'affection abdominale inflammatoire chez un mammifère avec de l'IL-11 humaine, comprenant l'administration de ladite protéine de fusion de l'albumine audit mammifère.
